(19) Europäisches Patentamt
European Patent Office
Office européen des brevets

(11) Publication number: 0 234 178 A1

# (12) EUROPEAN PATENT APPLICATION

(21) Application number: 86850438.2

(22) Date of filing: 15.12.86

(51) Int. Cl.4: **C07H 7/02** , C07H 9/04 , C07H 13/02 , C07H 15/04 , C07K 9/00 , A61K 31/70

The title of the invention has been amended (Guidelines for Examination in the EPO, A-III, 7.3).

(30) Priority: 20.12.85 SE 8506094

(43) Date of publication of application: 02.09.87 Bulletin 87/36

(84) Designated Contracting States: ES GR

(71) Applicant: **Astra Läkemedel Aktiebolag**
**Strängnäsvägen 44**
**S-151 85 Södertälje(SE)**

(72) Inventor: **Claesson, Alf**
**Lilldalsvägen 17A**
**S-144 00 Rönninge(SE)**
Inventor: **Hammond, Stephen Mark**
**Smedvägen 29**
**S-151 59 Södertälje(SE)**
Inventor: **Jansson, Anita Margareta**
**Fyrgränd 5**
**S-171 52 Solna(SE)**
Inventor: **Pring, Brian George**
**Västergardsvägen8**
**S-151 46 Södertälje(SE)**

(74) Representative: **Hjertman, Ivan T. et al**
**AB ASTRA Patent and Trade Mark Department**
**S-151 85 Södertälje(SE)**

## (54) New 8-substituted derivatives of an anhydrooctonic acid, methods for their preparation, pharmaceutical preparations containing these and intermediates.

(57) Novel compounds of the formula

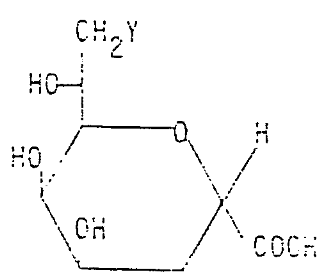

wherein Y is hydrogen, halogen, amino, acylamino, acyloxy, azido, mercapto, thiocarboxy, the groups

EP 0 234 178 A1

O-CHCOW (i) or -S-CHCOW (ii)
|   |
NHAA   NHAA

in which AA is an amino acid or peptide and W is OH or the same as AA; or physiologically acceptable salts thereof, processes for their preparation, pharmaceutical preparations containing the compounds, use of the compounds, and methods for treatment, and chemical intermediates. The compounds are active as enzyme inhibitors and useful as antibacterial agents or as intermediates for preparing antibacterial agents.

# New antibacterial agents and intermediates

## Field of the invention

The present invention relates to new 8-substituted derivatives of an anhydrooctonic acid and to methods for their preparation as well as to new intermediates useful for the preparation of 8-substituted anhydrooctonic acids. The acids are useful as inhibitors of bacterial lipopolysaccharide biosynthesis and hence as antibacterial agents or as intermediates for the production of antibacterial agents. The acids are also useful in that they may potentiate the action of other antibacterial agents. The invention also relates to the preparation of pharmaceutical preparations containing the said derivatives.

## Background of the invention

Lipopolysaccharides (LPS) form a unique class of macromolecules which are characteristic of Gram-negative bacteria. They constitute the outer part of the outer membrane of the microorganisms and are involved in the interaction of the cell with the environment. LPS thus plays a critical role in determining the antigenicity, toxicity, adhesiveness, invasiveness, and penetrability of the cell. The outer membrane constitutes an important structure of the Gram-negative cell by protecting it against the environment, e.g. the host defense system and antibacterial agents.

The structure of LPS in Salmonella tryphimurium is shown schematically in the Figure below.

(Tetrasaccharide)$_8$ ¦ Hex-Hex-Hep-Hep-KDO-KDO ¦ Lipid A

O-Antigen Core

The Lipid A constitutes the innermost part of LPS. Hex = Hexose, Hep = Heptose, KDO = 3-deoxy-D-manno-octulosonic acid.

Due to the importance of LPS for bacterial survival it might be expected that damage to it would affect viability. It has thus been found that mutants defective in 3-deoxy-D-manno-octulosonic acid (KDO) biosynthesis are no longer viable. Enzymes involved in biosynthesis of this acid might therefore constitute valuable targets for chemical compounds, which by inhibition of the enzymes would act as novel antimicrobial agents.

## Prior art

3-Deoxy-D-manno-2-octulosonic acid (KDO) is described in Advances in Carbohydrate Chemistry and Biochemistry, 38, 323-388 (1981).

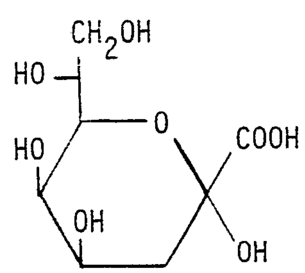

KDO

Some analogues of KDO are reported to exert an inhibitory effect upon the enzyme CMP-KDO synthetase - (p. 388).

Ray et al (ACS Symposium Series 231, 141-169) describes the synthesis of analogues of 3-deoxy-D-manno-2-octulosonic acid (KDO) and their use in inhibiting the biosynthesis of the LPS of Escherichia coli. Analogues of KDO have been synthesized and tested as inhibitors or alternative substrates for CMP-KDO synthetase. The substrate analogues were weak inhibitors of the enzymes involved in KDO biosynthesis.

In J. Med. Chem., 27, 717-26 (1984) compounds are described which are reported to inhibit arabinose-5-phosphate isomerase in the biosynthesis of lipopolysaccharide.

Techniques of exploiting the bacterial peptide transport system to bring normally impermeant compounds into the cell by linkage to a peptide were disclosed by Fickel and Gilvarg in Nature New Biology, 241, 161-163 (1973) and by Gilvarg et al in U.S. Patent 4,454, 065.

## Disclosure of the invention

The present invention relates to new compounds, having the formula I, which are 8-substituted derivatives of 2,6-anhydro-3-deoxy-D-glycero-D-talo-octonic acid and physiologically acceptable salts thereof, wherein Y may represent a member selected from the group consisting of hydrogen, halogen, amino, acylamino, acyloxy, azido, mercapto or thiocarboxy.

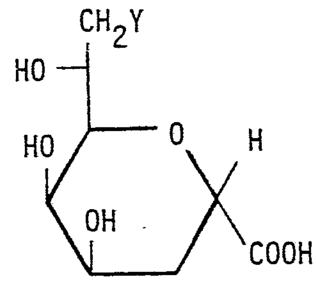

I

Thiocarboxy in formula I is defined as $R^a-\overset{O}{\overset{\|}{C}}-S-$ wherein $R^a$ and $R^a-\overset{O}{\overset{\|}{C}}-$ are as defined below.

Acylamino in formula I is defined as $R^a-\overset{O}{\overset{\|}{C}}-NH-$ wherein $R^a$ is an alkyl group containing 1-6 carbon atoms or a substituted or unsubstituted phenyl group or $R^a-\overset{O}{\overset{\|}{C}}-$ is an amino acid or an amino acid sequence containing 2-4 amino acid residues. Preferably the amino acids can be optionally chosen from the group consisting of glycyl, alanyl, arginyl, phenylalanyl, histidyl, lysyl, ornithyl, norvalyl, valyl, norleucyl, isoleucyl, leucyl, methionyl, prolyl or 2-aminopimelyl.

Halogen in formula I is defined as Cl, F, Br or I. Acyloxy in formula I is defined as $R^a-\overset{O}{\overset{\|}{C}}-O-$, wherein $R^a$ is as defined above or $R^a-\overset{O}{\overset{\|}{C}}-$ is an amino acid or an amino acid sequence containing 2-4 amino acid residues. The amino acids can be chosen from the group mentioned above.

Y can also be a group

$$-O-\underset{\substack{|\\ NH-AA}}{CH}COW \quad (i) \qquad \text{or} \qquad -S-\underset{\substack{|\\ NH-AA}}{CH}COW \quad (ii)$$

wherein AA is an amino acid or an amino acid sequence containing 2-4 amino acid residues, preferably chosen from the group above either in the L-or in the D-configuration and W is hydroxy, amino, benzyloxy or an amino acid or amino acid sequence containing 2-4 amino acid residues chosen from the group above either in the L-or in the D-configuration.

It has surprisingly been found that the compounds of the invention exhibit strong inhibitory activity on the enzyme cytidine monophosphate 3-deoxy-D-manno-2-octulosonate synthetase (CMP-KDO synthetase) or within the bacterial cell can be converted to such inhibitors, or are useful as intermediates for the preparation of compounds exhibiting inhibitory activity on CMP-KDO synthetase. In certain cases, where the group Y represents a member selected from the group consisting of acylamino and the groups (i) and -(ii), the compounds may be particularly readily transported through the bacterial cell wall and either by direct action or after transformation exert an inhibitory action on CMP-KDO synthetase thereby preventing biosynthesis of lipopolysaccharide (LPS) and resulting in an antibacterial effect either alone or in the presence of certain agents which do not normally have a strong antibacterial effect on the intact bacterial cells. As a result of this effect, administration of such compounds or certain mixtures containing such compounds to an infected host is of value in the therapy of bacterial infections caused by Gram-negative bacteria.

Preferred compounds of this invention are those having the following formulas (all amino acids have the L-configuration):

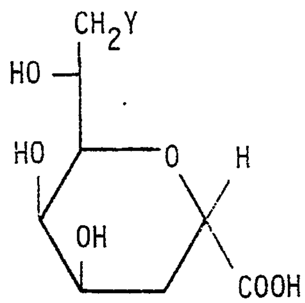

Y

H,
$N_3$,
$NH_2$,
NH-Ala,
S-Ala-Ala,
NH-Ala-Met,
NH-Ala-Ala,
NH-Ala-Phe,

Y cont.

NH-Arg-Ala,
NH-Met-Val,
SH,
F,
NH-Met-Pro,
NH-Ala-Lys,
NH-Ala-His
NH-Ala-Val,
NH-Ala-Leu,
NH-Nva-2-aminopimelyl,
NH-Ala-Orn,
NH-Nva-Arg,
NH-His-Ala,
NH-Lys-Ala,
NH-2-aminopimelyl-Arg
O-Ala-Ala,
O-Ala-His,
O-Ala-Lys,
O-His-Ala,
O-Lys-Ala,

```
Ala-NH-CHCOOH,
       |
       -O
Ala-Ala-NH-CHCOOH,
           |
           -O
Lys-Ala-NH-CHCOOH,
           |
           -O
Ala-NH-CHCOOH,
       |
       -S
Ala-Ala-NH-CHCOOH,
           |
           -S
Lys-Ala-NH-CHCOOH,
           |
           -S
```

## Methods of preparation

The compounds of this invention are most conveniently prepared by removal of protective groups from a compound of the formula

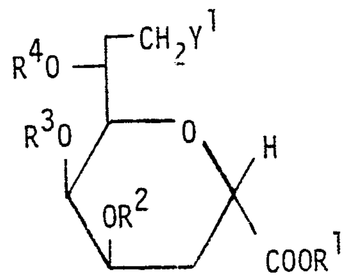

wherein $R^1$-$R^4$ are the same or different and selected from hydrogen, or a protecting group known in the art (see for example T.W. Greene, "Protective Groups in Organic Chemistry" Wiley I98I);
$Y^1$ is defined as Y or a group $-N(Y^2)_n$, wherein n is I or 2 and $Y^2$ is allyl, benzyl, tert-butoxycarbonyl, trifluoroacetyl or arenesulfonyl; phthalimido; an amino group NH-Q-Z, wherein Q and Z are defined as below; thiocarboxy; acyloxy -O-Q-Z, wherein Q and Z are as defined below; a group

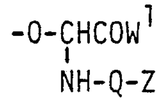 or 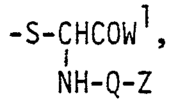,

wherein $W^1$ is amino, benzyloxy or a protected amino acid or peptide sequence containing 2-4 amino acid residues and Q and Z are as defined below;
Q is a group containing from I-4 residue units of an amino acid such as histidyl, glycyl, alanyl, phenylalanyl, lysyl, ornithyl, norvalyl, valyl, norleucyl, isoleucyl, leucyl, methionyl, prolyl, arginyl or 2-amino-pimelyl either in the natural L-or in the D-configuration which is protected as is conventional in the peptide art;
Z is an amino protecting group which is used in the polypeptide art. Such groups may be those removable either by selective hydrolysis or exchange hydrogenolysis over a noble metal, such as palladium, in the presence of a hydrogen donnor. Examples of these groups are aralkyl or alkyloxycarbonyl groups, for example; trichloroethoxycarbonyl, allyloxycarbonyl, benzyloxycarbonyl or tert-butoxycarbonyl. A protecting group which can be removed by the same chemical reaction as the groups $R^1$-$R^4$ is preferred.
$R^2$-$R^4$ may be the same or different and selected from protective groups such as acyl, aralkyl such as benzyl, allyl, tetrahydropyranyl or substituted silyl, or cyclic acetals such as acetonides used for vicinal diols. Examples of protective groups $R^1$ are $C_1$-$C_6$ alkyl, benzyl or allyl. The protected forms of the claimed compounds may be obtained by the following methods constituting a further aspect of the invention.

Method A. Reacting a protected compound having a suitable leaving group X, with a nucleophilic reagent, which delivers the group $Y^1$.

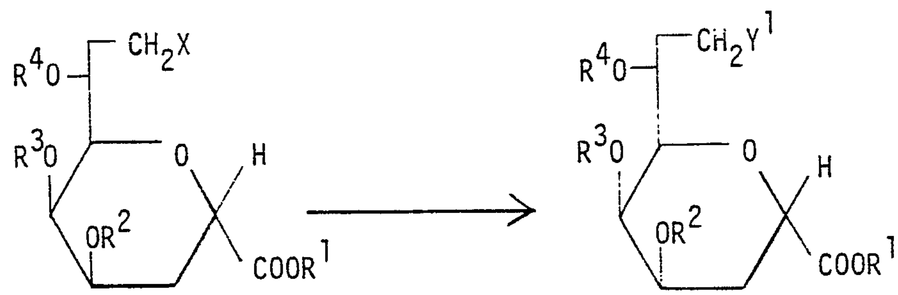

The $R^1$-$R^4$ groups are as described above. X is a leaving group known to those skilled in the art e.g. a group such as iodo, bromo or a group $OSO_2R^5$ where $R^5$ is alkyl containing I-8 carbon atoms, perfluorinated alkyl containing I-8 carbon atoms or aryl. The nucleophilic reagent may be chosen among a variety of salts or salt-like compounds $MY^1$ where M is a mono-or divalent metal ion such as $Na^+$ or $K^+$ or a group $(R^6)_4N^+$, -$(R^6)_3NH^+$ or $(R^6)_4P^+$ where $R^6$ is phenyl or alkyl containing I-I8 carbon atoms and $Y^1$ is an anion such as fluoride, chloride, azide, $SH^-$, $R^7COS^-$ where $R^7$ is alkyl containing I-8 carbon atoms or aryl, thiocyanate,

trifluoroacetamido, sulphonamido. Examples of such salts are KF, $(C_4H_9)_4NF$, sodium azide, magnesium chloride, triethylammonium hydrogen sulphide, sodium thiobenzoate, trifluoroacetamide sodium salt. The nucleophilic reagent can also be ammonia or a primary amine ($R^8NH_2$) or secondary amine $(R^8)_2NH$, where $R^8$ is benzyl or allyl.

The reaction is preferably conducted in an organic solvent such as for example dimethylformamide, acetonitrile, 2-propanol or tetrahydrofuran, at a temperature of between 0-70°C for 5 min to 3 days.

After substitution the protective groups $R^1$-$R^4$ are hydrolyzed with base such as sodium hydroxide l-5 M in water or methanol when $R^2$-$R^4$ are acyl, or hydrogenated in an organic solvent such as for example tetrahydrofuran water or ethanol over a metal catalyst for l-24 h at a pressure of l00-5000 kPa (l-50 atmospheres). The hydrogenation also converts an azido group into an amino group and hydrogenolyzes a halide which may be taken advantage of in the preparation of the end products. When $R^2$-$R^4$ are silyl-containing groups the compound is hydrolyzed with tetrabutylammonium fluoride or hydrogen fluoride. When $R^1$ is an acid labile group, such as for example triarylmethyl, this group may be removed by treatment with trifluoroacetic acid.

When $Y^1$ is thiocyano the hydrolysis may be performed with a reagent such as mercuric chloride in a separate step. When $Y^1$ is azido and $R^1$-$R^4$ are protective groups stable towards amines, reduction can be performed stepwise starting by a treatment with a reducing reagent such as hydrogen sulphide or a trivalent phosphorous reagent such as triphenylphosphine or triethyl phosphite.

Method B. Reacting a hydroxy-ester,

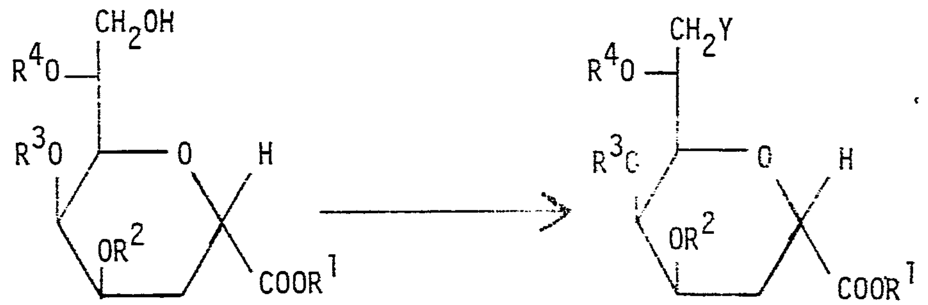

where the other hydroxy groups may optionally be protected with a phosphorus-or sulfur-based electrophilic reagent.

$R^1$-$R^4$ are as defined under A above and Y is fluoro, chloro, thiocarboxy or azido.

The reaction may be performed with a fluorine-delivering reagent such as sulfur tetrafluoride or preferably dialkylaminosulfur trifluorides such as diethylaminosulfur trifluoride (DAST). Phosphorus-based reagents, for example such as those described in "Organophosphorus Reagents in Organic Synthesis" (J.I. Cadogan, Ed.) Academic Press l979, are well suited for replacement of a hydroxy group by a halogen, azido or thiocarboxy group.

On using these reagents the hydroxyl groups need not be protected i.e. $R^2$-$R^4$ are hydrogens. Examples of reagents of this type are the combinations of triphenylphosphine, tetrabromomethane and lithium azide as described by Yamamoto et al (J.C.S., Perkin I, l980, 306) or of triphenylphosphine, diethyl azodicarboxylate and ethanethioic acid as described by Jenkins and Thang (Aust. J. Chem. l984, 37, l925).

The reaction is performed in an organic solvent such as for example dimethylformamide or tetrahydrofuran. The protective groups are then removed as described in Method A.

Method C. Reacting an aldehyde, wherein $R^1$-$R^4$ are protective groups as described above with

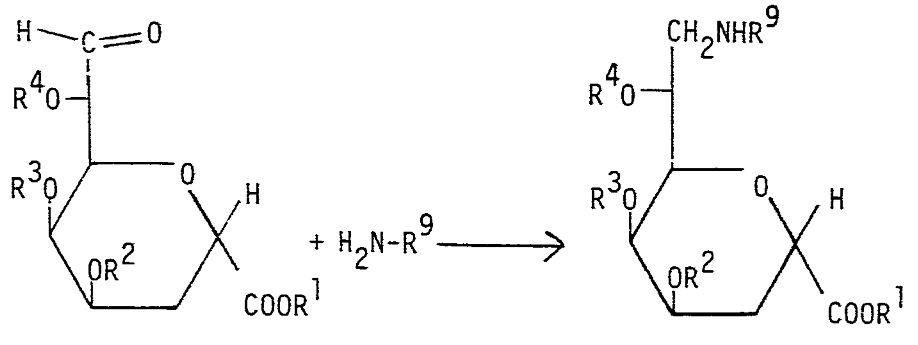

an amine, wherein $R^9$ is hydrogen, benzyl or allyl, under reducing conditions. Some examples of reagents that may be used are sodium cyanoborohydride, sodium borohydride or hydrogen in the presence of a noble metal catalyst.

The reductive amination is preferably conducted in an organic solvent such as ethanol, tetrahydrofuran or dimethylformamide.

Method D. Reaction of an amino acid or oligopeptide with an amine

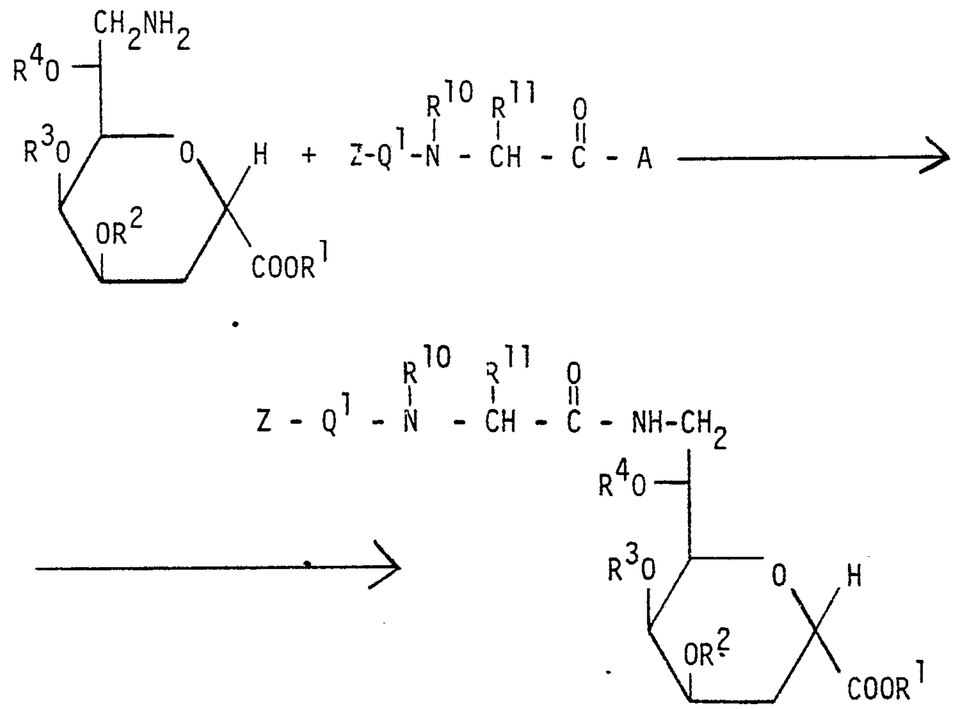

wherein $R^1$-$R^4$ are protective groups as described above;

$Q^1$ is a group containing from O-3 residue units of an amino acid such as glycyl, alanyl, phenylalanyl, lysyl, ornithyl, histidyl, norvalyl, arginyl, valyl, norleucyl, isoleucyl, leucyl, methionyl, prolyl or 2-aminopimelyl, either in the natural L-or in the D-configuration, which is protected as is conventional in the peptide art, whereby the amino acid units are the same or different;

Z is an amino protecting agent which is used in the polypeptide art. Such groups may be those removable either by selective hydrolysis or exchange hydrogenolysis over a noble metal, such as palladium, in the presence of a hydrogen donnor. Example of these groups are aralkyl or alkoxycarbonyl groups, for example; trichloroethoxycarbonyl, allyloxy, benzyloxycarbonyl or tert-butoxycarbonyl. A protective group which can be removed by the same chemical reaction as the groups $R^1$-$R^4$ are preferred.

$R^{10}$ is $C_{1-4}$ lower alkyl, or hydrogen, $R^{11}$ is $C_{l-4}$ lower alkyl, phenyl, $\omega$-amino $C_{1-4}$ alkyl, benzyl or 2-thioethyl. $R^{11}$ may also be connected to $R^{10}$ to form a cyclic derivative in which $R^{10}$ and $R^{11}$ together form a chain containing from 2-5 carbon atoms.

A is hydroxy or a carboxyl-activating group as is used in the peptide art (see for example Houben-Weyl "Methoden der Organischen Chemie", Band l5/l and l5/2). Examples of such activating groups are substituted aryl, such as pentachlorophenoxy or 4-nitrophenoxy, imidoxy such as succinimidoxy.

Method E. Reaction of an amino acid or oligopeptide with an azide.

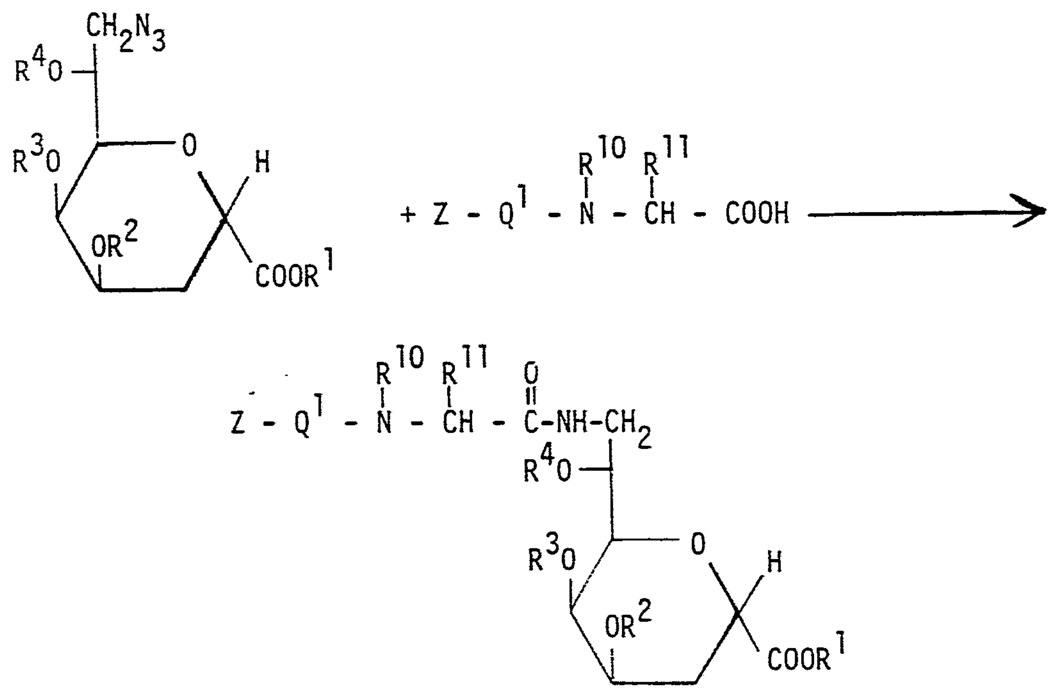

wherein $R^1$-$R^4$ are protective groups as described above and Z, $Q^1$, $R^{10}$ and $R^{11}$ are as described under D.

The reaction is performed in a nonprotic organic solvent such as toluene, dichloromethane or acetonitrile by treatment of the azide, before or after addition of the acid, with a compound $(R^{12})_3P$, wherein $R^{12}$ may be alkyl ($C_1$-$C_8$), aryl, or alkoxy ($C_1$-$C_4$) or aryloxy. Reaction temperature may vary between room temperature and 120°C. The principles of this reaction is described by Zaloom et al. J. Org. Chem. 1985, 50, 2601.

Method F. Reacting a hydroxy-ester or a thiol,

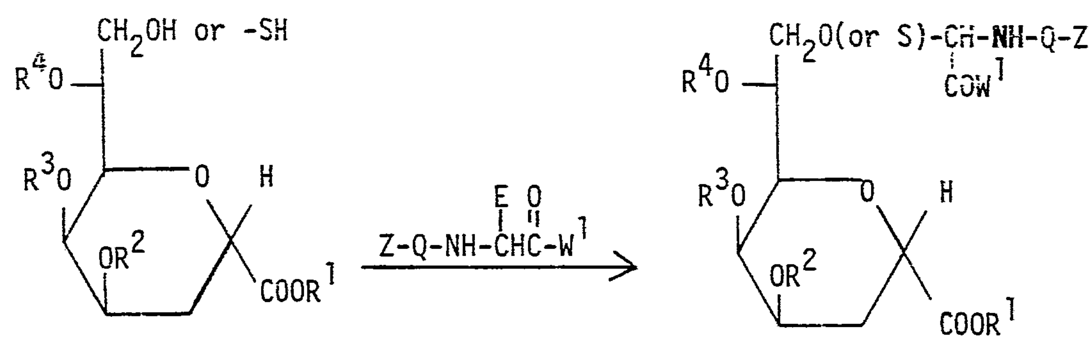

wherein the non-reacting hydroxy groups may optionally by protected with a glycine derivative, such as is described in U.S. Patent 4,454,065, under the conditions described in the same publication.
E is a leaving group such as chloride, acetoxy, or mesyloxy;
$R^1$-$R^4$, Q, Z, and $W^1$ are as defined above.

Preparation of starting materials

The peptides used as one of the reactants in Methods D and E are either known compounds or can be prepared by methods known to those skilled in the art.

The anhydrooctonic acid derivatives used in methods A-F are all new compounds, the preparation of which constitute a valuable aspect of the invention. The following ester, wherein $R^1$ is a protecting group such as $C_1$-$C_5$ lower alkyl, allyl or benzyl, is a preferred intermediate.

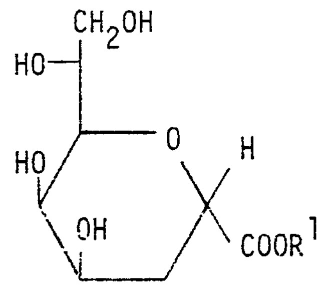

(V)

This ester (V) is then carried through a series of steps, in many cases involving protection of the three non-participating hydroxyl groups, intended to give the preferred compounds of this invention. It can be prepared along three different routes which are here called methods G-I.

Method G. Reduction of a KDO derivative of the formulas IIA or IIB, followed by removal of protecting groups

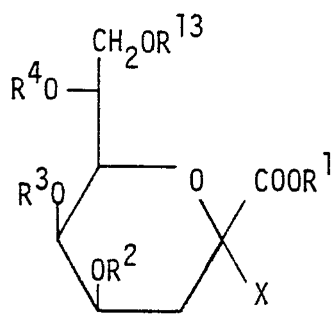

IIA

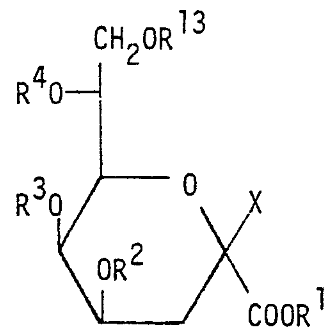

IIB

wherein X is a halogen atom, preferably chloro or bromo, or a sulfur-containing group such as alkylthio, arylthio or aryl-SCOO or thio-carbonyl, $R^2$-$R^4$ and $R^{13}$ are hydrogen, acyl, aralkyl such as benzyl, 2-tetrahydropyranyl, cyclic acetal such as acetonide or a protecting group known in the art. $R^1$ is hydrogen, an alkyl group containing l-6 carbon atoms, aralkyl such as benzyl, aryl or a protecting group known in the art. A great variety of protecting groups are known to those skilled in the art by for instance "Protecting Groups in Organic Chemistry" T.W. Greene, Wiley l98l.

The reduction is preferably carried out using hydrogen in combination with a metal catalyst such as palladium, platina or nickel. Alternatively, the reduction can be carried out under radical generating conditions involving a hydride reducing agent such as trialkyltin hydride.

The reduction with hydrogen may be performed in a suitable solvent, such as ethyl acetate, toluene, acetone or dimethyl formamide (DMF) at a temperature between O°C and 5O°C in the presence of a tertiary amine such as pyridine or triethylamine.

Method H. Epimerization (base-catalyzed isomerization) of a compound of the formula

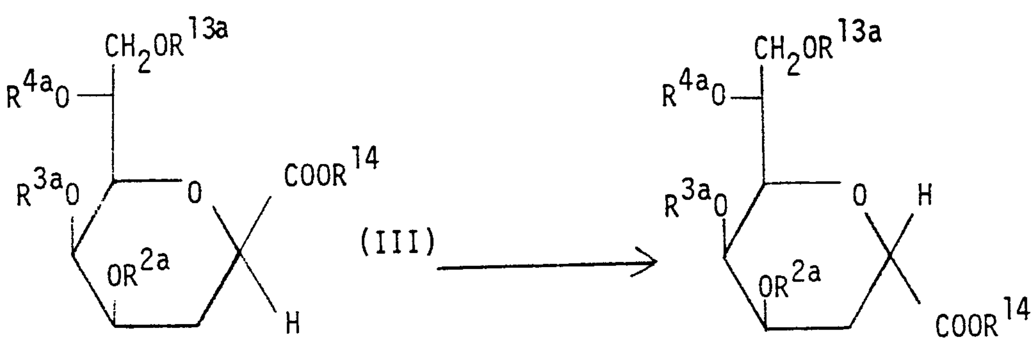

(III)

followed by removal of protecting groups
wherein $R^{2a}$-$R^{4a}$ and $R^{13a}$ are hydrogen or chosen among the protective groups which are stable towards base such as aralkyl or cyclic acetal, preferably benzyl or acetonide and $R^{14}$ is an alkyl group. The epimerization may be performed in an alcoholic solvent e.g. methanol or ethanol containing a metal alkoxide corresponding to $R^{14}$ or by treatment with a metal amide such as lithium diisopropylamide in a suitable

solvent such as tetrahydrofuran or diethyl ether.

Method I. Cyclization of a partially protected compound of the formula

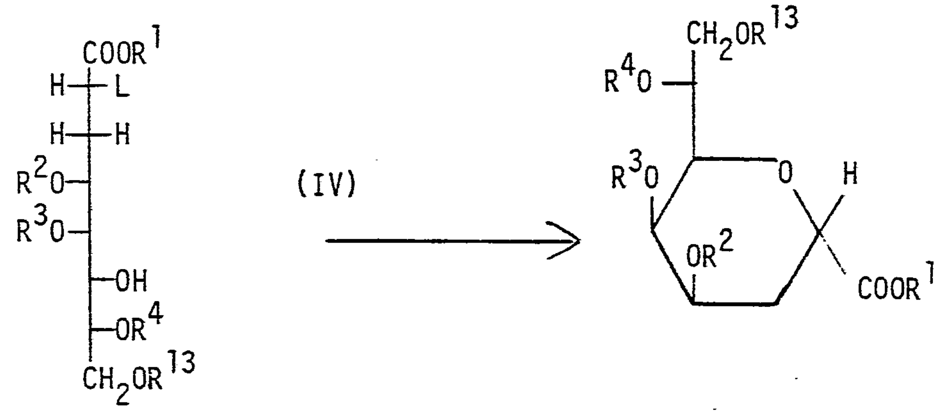

wherein L is a leaving group e.g. a halogen atom such as chloro, bromo or iodo, a sulfonate group, or a phosphoniooxy group $(R^{15})_3\overset{+}{P}O$-, where $R^{15}$ is phenyl or alkyl containing I-4 carbon atoms. $R^1$-$R^4$ and $R^{13}$ protecting groups defined under G above. The cyclization is initiated by adding a base such as potassium carbonate or metal alkoxide to a solution of the compound of formula IV in an alcoholic solvent, e.g. methanol or ethanol or a mixture including said alcohols, at room temperature to 5O°C or by treatment of the compound IV with sodium hydride in an inert solvent such as tetrahydrofuran or toluene.

The protecting groups of the claimed acid may be removed by one or more chemical reactions known to those skilled in the art e.g. hydrolysis, to obtain the compound of the formula I.

Salts

Physiologically acceptable salts of compounds of the invention are prepared by methods known in the art. The salts are novel compounds and comprise a further aspect of the invention. Addition salt can be prepared by treating the compound of the invention with for example ammonia. Metal salts can be prepared by treating a metal hydroxide with a compound of the invention. Examples of metal salts which can be prepared in this way are salts containing Li, Na, and K. A less soluble metal salt can be precipitated from a solution of a more soluble salt by addition of a suitable metal compound.

Pharmaceutical preparations

Pharmaceutical preparations of the compound of the invention constitute a further aspect of the invention.

The compounds of the invention can be administered systemically or locally and can be administered topically, orally, by the rectal route, by injection, by infusion, or by inhalation in the form of a pharmaceutical preparation comprising the active ingredient in the form of the original compound or optionally in the form of a pharmaceutically acceptable salt thereof, in association with a pharmaceutically acceptable carrier which may be a solid, semi-solid or liquid diluent or an ingestible capsule, and such preparations comprise a further aspect of the invention. The compounds may also be used without carrier material. As examples of pharmaceutical preparations may be mentioned tablets, solutions, drops, such as nasal drops, eye drops, preparations for topical application such as ointments, jellies, creams and suspension, aerosols for inhalation, nasal spray, liposomes, etc. Usually the active substance will comprise between O.OI and 99, or between O.I and 99% by weight of the preparation, for example between O.5 and 2O% for preparations intended for injection and between O.I and 5O% for preparations intended for oral administration.

The preparations are preferably in dosage unit form. Further, they are preferably provided in sterilized form.

To produce pharmaceutical preparations in the form of dosage unit for oral application containing a compound of the invention the active ingredient may be mixed with a solid, pulverulent carrier, for example lactose, saccharose, sorbitol, mannitol, a starch such as potato starch, corn starch, amylopectin, laminaria powder or citrus pulp powder, a cellulose derivative or gelatine and also may include lubricants such as

magnesium or calcium stearate or a Carbowax® or other polyethylene glycol waxes and compressed to form tablets or cores for dragées. If dragées are required, the cores may be coated for example with concentrated sugar solutions which may contain gum arabic, talc and/or titanium dioxide, or alternatively with a film forming agent dissolved in easily volatile organic solvents or mixtures of organic solvents. Dyestuffs can be added to these coatings, for example, to distinguish between different contents of active substance. For the preparation of soft gelatine capsules consisting of gelatine and, for example, glycerol and a plasticizer, or similar closed capsules, the active substance may be admixed with a Carbowax® or a suitable oil as e.g. sesame oil, olive oil, or arachis oil. Hard gelatine capsules may contain granulates of the active substance with solid, pulverulent carriers such as lactose, saccharose, sorbitol, mannitol, starches (for example potato starch) corn starch or amylopectin), cellulose derivatives or gelatine, and may also include magnesium stearate or stearic acid as lubricants.

By using several layers of the active drug, separated by slowly dissolving coatings sustained release tablets are obtained. Another way of preparing sustained release tablets is to divide the dose of the active drug into granules with coatings of different thicknesses and compress the granules into tablets together with the carrier substance.

The active substance can also be incorporated in slowly dissolving tablets made for instance of fat and wax substances or evenly distributed in a tablet of an insoluble substance such as physiologically inert plastic substance.

Liquid preparations for oral application may be in the form of elixirs, syrups or suspensions, for example solutions containing from about 0.1% to 20% by weight of active substance, sugar and a mixture of ethanol, water, glycerol, propylene glycol and optionally aroma, saccharine and/or carboxymethylcellulose as a dispersing agent.

For parenteral application by injection preparations may comprise an aqueous solution of the active drug or a physiologically acceptable salt thereof, desirably in a concentration of 0.05-10%, and optionally also a stabilizing agent and/or buffer substances in aqueous solution. Dosage units of the solution may advantageously be enclosed in ampoules.

The dosage at which the active ingredients are administered may vary within a wide range and will depend on various factors such as for example the severity of the infection, the age of the patient, etc., and may have to be individually adjusted.

The pharmaceutical compositions containing the active ingredients may suitably be formulated so that they provide doses within these ranges either as single dosage units or as multiple dosage units.

Intermediates

Most of the intermediates or starting materials used in the methods A-F are novel and constitute a further aspect of the invention. Thus, in one aspect the invention is related to novel compounds of the formula

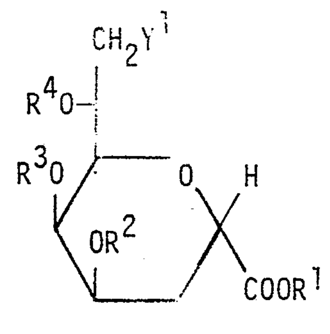

wherein $R^2$-$R^4$ are the same or different and selected from hydrogen or protecting groups such as tert. butyldimethyl-silyl, benzyl;

$R^1$ is a protecting group such as methyl, benzyl or allyl;

$Y^1$ is hydrogen, a halogen, such as fluoro or chloro, amino, azido, mercapto, acylamino, thiocarboxy, acyloxy, Y or a group $N(Y^2)_n$, wherein n is 1 or 2 and $Y^2$ is allyl, benzyl, tert.-butoxycarbonyl, trifluoroacetyl or arenesulfonyl; phthalimido; thiocarboxy; acyloxy -O-Q-Z wherein Q and Z are defined on page 6; an amino group NH-Q-Z, wherein Q and Z are defined on page 6 ; a group

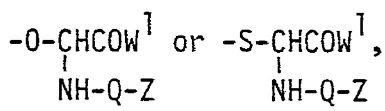

wherein W' is amino, benzyloxy or a protected amino acid or peptide sequence containing 2-4 amino acid residues and Q and Z are defined on page 6.

Preferred intermediates are those wherein Y' is hydrogen, halogen, amino, azido, mercapto, acylamino, acyloxy or thiocarboxy.

Especially preferred intermediates are compounds of the formula

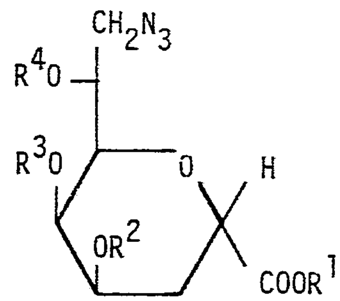

wherein $R^2$-$R^4$ are the same or different and selected from hydrogen or protecting groups such as tert.-butyldimethylsilyl or benzyl;
$R^1$ is a protecting group such as methyl, benzyl or allyl.

Biological tests

The inhibitory effect of the compounds of the invention on Gram-negative bacteria and in isolated enzyme systems was tested using the methods described below.

In the following the compound N-(L-alanyl-L-alanyl)-8-amino-2,6-anhydro-3,8-dideoxy-D-glycero-D-talo-octonic acid is denoted compound 6.

Test I Bactericidal activity

A range of Gram-negative bacteria were grown at 37°C in aerobic shake culture in nutrient broth for 16 h. 0.1 ml of these cultures were each inoculated into 5 ml of fresh broth and the cultures incubated further, until the bacteria reached mid-logarithmic phase, when the culture was harvested by centrifugation and resuspended in Basal Medium 2 (J Bacteriol 117 (1974) 302-311) to an OD $^{1cm}_{620}$ mm = 0.2 (equivalent to $10^8$ bacteria/ml). Test compound was added to the bacterial suspension to give a final concentration of 1mM. After 15 min exposure to drug the number of viable bacteria was determined by serial dilution in sterile phosphate buffered saline (PBS), plating out onto McConkey agar plates and incubating for 16 h at 37°C. The results are shown in Table I.

The compound N-(L-alanyl-L-alanyl)-8-amino-2,6-anhydro-3,8-dideoxy-D-glycero-D-talo-octonic acid - (Compound 6) was found to have inhibitory action against the bacteria.

Table 1. Percentage surviving bacteria after exposure of bacterial suspension to 1mM KDO inhibitors

| | Salmonella typhimurium SH 6749 | Escherichia coli C10 | Klebsiella aerogenes NCTC 1228 | Proteus mirabilis 1790 |
|---|---|---|---|---|
| No drug control | 100% | 100% | 100% | 100% |
| 1 mM Compound 6 | 0.4% | 0.01% | 2.8% | 55% |

Test II Minimum Inhibitory Concentration (MIC) and Minimum Bactericidal Concentration (MBC) Determination

Two fold serial dilutions of the test compound were made in a defined minimal medium such as Davies Minimal Medium (Difco) in the wells of a Microtitre plate, giving a final volume of IOO µl/well. $IO^3$ bacteria, from an overnight bacterial culture, were inoculated into each well and the Microtitre plate incubated at 37°C overnight. The MIC was judged to be the lowest drug concentration at which no turbidity could then be detected.

To determine the minimum bacterial concentration (MBC) I µl aliquots from each well of the MIC plate (after overnight incubation) were aseptically transferred by replica plating onto a blood agar plate (Blood agar base (Difco) plus 5% horse blood), which was incubated overnight at 37°C. The MBC was judged to be the lowest drug concentration from which no bacterial colonies appeared on the blood agar plate after incubation.

MIC and MBC values of KDO analogues are shown in Table 2.

Table 2. MIC and MBC values (μmol) of KDO analogues

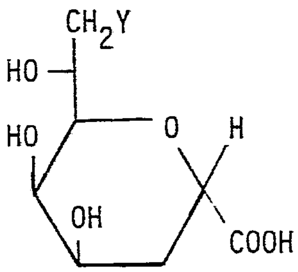

| Y | Salmonella SL 1102 MIC | Salmonella SL 1102 MBC | Salmonella LT2 M-I MIC | Salmonella LT2 M-I MBC | Escherichia ATCC 6-11303 MIC | Escherichia ATCC 6-11303 MBC | Salmonella typhimurium AG701 i50 MIC | Salmonella typhimurium AG701 i50 MBC |
|---|---|---|---|---|---|---|---|---|
| Val-Met-NH- | 16 | 16 | 16 | 60 | 3.9 | 3.9 | 1.9 | 1.9 |
| Ala-Ala-Ala-NH- | >1500 | | >1500 | | 50 | 90 | 12 | 12 |
| Leu-Leu-NH- | 250 | 500 | 2 | 2 | 0.5 | 0.5 | 3.9 | 3.9 |
| Pro-Met-NH- | 16 | 30 | 30 | 60 | 30 | 30 | <0.9 | |
| Ala-Phe-NH- | 250 | 250 | 120 | 250 | 8 | 16 | <0.9 | |
| Ala-Arg-NH- | > 2 | | > 2 | | 8 | 8 | NT | |
| Lys-Leu-NH- | 30 | 30 | 30 | 125 | 8 | 16 | NT | |
| Arg-Nva-NH- | 2 | 8 | > 2 | | 1 | 2 | NT | |
| N-Me-Ala-Nva-NH- | 60 | 250 | > 2 | | 250 | 500 | NT | |
| Ala-Ala-NH- (compound 6) | 4 | NT | 2 | NT | 4 | NT | | NT |

NT = not tested

Test III Inhibition of incorporation of $^3$H-arabinose-5-phosphate into LPS

Arabinose-5-phosphate is a precursor of KDO in enteric bacteria. KdsA mutants of Salmonella typhimurium rapidly take up exogenous arabinose-5-phosphate and convert it to KDO which then appears exclusively in LPS (J. Biol. Chem. 255 (1980) 4257).

S. typhimurium AG701 i50 ($KdsA^{ts}$) was grown overnight at 30°C in minimal Medium (Eur. J. Biochem. 107 (1980) 145-153) supplemented with 1% glycerol and 0.01 mM D-fucose. The next day 0.1 ml of overnight culture was added to fresh medium and the culture grown at 30°C until mid logarithmic phase - (OD $_{620}^{1cm}$ mm = 0.2) when 50 µl of $^{3}$H-arabinose-5-phosphate (Amersham; specific activity 72 µCi/ml)- was added. After 10 min 0.45 ml samples were taken, the bacteria colleced by filtration (Whatman GF/F glass fibre filter) and washed with 6 x 1ml PBS followed by 100 ml boiling water. This procedure ensures that all radioactive counts recorded represent arabinose-5-phosphate incorporated into LPS as KDO (J. Biol. Chem. 255 (1980) 4257). The results are shown in Table 3.

It was observed that Compound 6 has an effect upon the incorporation of arabinose-5-phosphate into LPS and that it markedly inhibits denovo LPS biosynthesis.

Table 3. Inhibition of denovo LPS biosymthesis by KDO analogues as measured by incorporation of arabinose-5-phosphate into LPS

| | Arabinose-5-phosphate incorporated into LPS (µmoles/mg dry wt bacteria) | % inhibition |
|---|---|---|
| Control | 1.1 | - |
| 0.1 mM Compound 6 | 0.56 | 49% |
| 1 mM Compound 6 | 0.5 | 65% |

Test IV Synergy Testing

The outer membrane of Gram-negative bacteria is normally capable of excluding toxic dyes and certain antibiotics. E. coli ACTC 6-11303 grows normally on Antibiotic Assay Agar N °1 (Difco) supplemented with 20 µg crystal violet/ml or 200 µg spiramycin/ml. However, in the presence of agents which damage the outer membrane perameability barrier, sensitivity to agents such as spiramycin and crystal violet increase markedly.

5 ml of bacterial suspension (~$10^{6}$ bacteria/ml) was poured onto a sterile petri dish containing antibiotic assay medium of the same medium supplemented with 20 µg crystal violet/ml (or 200 µg spiramycin/ml). After 30 s the suspension was removed by aspiration and the plate dried at 37°C for 30 min. Wells were cut into the plate, each filled with 100 µl of test substances, the substances allowed to diffuse at room temperature for 30 min and the plate incubated overnight at 37°C. Next day the size of any inhibition zones around the wells were measured.

Synergy was judged to be demonstrated where the zone sizes on crystal violet (or spiramycin) plates were greater than those elicited by a similar concentration of drug on unsupplemented antibiotic assay agar. The results are shown in Table 3.

Table 4. Synergy between KDO analogues and agents that normally fail to penetrate the bacterial outer membrane

| | Zone diameter (mm) | | |
|---|---|---|---|
| | Control | Crystal Violet | Spiramycin |
| 2 μmoles Compound 6 | 0 | 20 | 16 |

<u>Test V Enzyme Inhibition Studies</u>

Potential inhibitors were tested for inhibitory action against two key enzymes involved in the biosynthesis of bacterial LPS.

I CMP-KDO synthetase (CKS) which catalyses the reaction

$$KDO + CTP \xrightarrow{CKS} CMP\text{-}KDO + PPi$$

2 CMP-KDO-lipid A transferase (KLT) which transfers CMP-KDO to an intermediate in the biosynthesis of LPS known as lipid A precursor

$$CMP\text{-}KDO + \text{lipid A precursor} \xrightarrow{KLT} KDO\text{-}\text{lipid A precursor} + CMP$$

CKS assay:

The CKS enzyme was prepared essentially as described by Osborn (J. Biol. Chem. <u>252</u> (1978) 3904-4903) and the enzyme assayed by the method of Ghalambor and Heath (J. Biol. Chem.) <u>241</u> (1966) 3216-3221).

KLT assay:

The KLT enzyme was purified essentially by the method of Osborne (J. Biol. Chem. <u>252</u> (1977) 1503-1511) and lipid A precursor by the method of Rick (J. Biol. Chem. <u>252</u> (1977) 4895-4903). KLT activity was determined by monitoring the incorporation of [$^{14}$C]-labelled CMP-KDO into acid-insoluble product. The radiolabelled CMP-KDO was generated <u>in situ</u> from CTP and[$^{14}$]-KDO by the action of added CKS enzyme. The incubation mixture contained CKS enzyme, KLT enzyme, 100 mM Tris buffer pH 8.0, 10.6 mM $MgCl_2$, 0.5 mM CTP, 0.4% Alfonic 1012-6, 0.16mM [$^{14}$C]-KDO and lipid A precursor. The final reaction volume was 150 μl. The mixture was incubated at 25°C for 15 min, when 50 μl was transferred to a filter paper circle - (Munktell No. 5, 24 mm) and then washed copiously with 10% TCA. The radio-activity adsorbed to the paper circle, which represents radiolabelled KDO bound to precursor was determined using liquid scintillation counting.

The results of the inhibition of KLT enzymes are shown in Table 5. The results of the inhibition of CKS enzymes are shown in Table 6. The inhibitory action is expressed in percent of a no inhibitor control run simultaneously.

Table 5. Percentage of inhibition of KLT enzymes by the following compounds

| Konc. (mM): | 0.64 | 0.16 | 0.04 | 0.01 | 0.0025 | 0.00625 |
|---|---|---|---|---|---|---|
| Compound 1 | 96 | 93 | 81 | 62 | 37 | 15 |
| Compound 2 | 97 | 93 | 83 | 57 | 28 | 14 |
| Compound 3 | 96 | 92 | 89 | 87 | 45 | |
| Compound 4 | 89 | 69 | 36 | 13 | 0 | |
| Compound 5 | 3 | 3 | 0 | | | |
| Compound 6 | 2 | 2 | | | | |
| Compound 7 | 95 | 87 | 62 | 36 | 10 | |

Table 6. Percentage of inhibition of CKS enzymes by the following compounds

| Konc. (mM): | 10.16 | 2.54 | 0.64 | 0.16 | 0.04 | 0.01 | 0.0025 |
|---|---|---|---|---|---|---|---|
| Compound 1 | 98 | 99 | 96 | 88 | 63 | 28 | 6 |
| Compound 2 | 100 | 96 | 96 | | | | |
| Compound 3 | 98 | 96 | 96 | 88 | 64 | 29 | 10 |
| Compound 4 | 99 | 95 | 90 | | | | |
| Compound 5 | 83 | 54 | 25 | 10 | 7 | | |
| Compound 6 | 66 | 39 | 13 | | | | |
| Compound 7 | 90 | 97 | 97 | 83 | 47 | 11 | |

Compound I = Ammonium 2,6-anhydro-3,8-dideoxy-8-fluoro-D-glycero-D-talo-octonate
Compound 2 = 8-amino-2,6-anhydro-D-glycero-D-talo-octonic acid
Compound 3 = Ammonium 2,6-anhydro-3,8-dideoxy-D-glycero-D-talo-octonate
Compound 4 = Ammonium 8-azido-2,6-anhydro-3,8-dideoxy-D-glycero-D-talo-octonate
Compound 5 = N-(L-alanyl)-8-amino-2,6-anhydro-3,8-dideoxy-D-glycero-D-talo-octonic acid
Compound 6 = N-(L-alanyl-L-alany)-8-amino-2,6-anhydro-3,8-dideoxy-D-glycero-D-talo-octonic acid
Compound 7 = Ammonium 2,6-anhydro-3,8-dideoxy-8-thio-D-glycero-D-talo-octonate

Working Examples

The following examples illustrate the preparation of compounds according to the invention.

The following general procedures were used for deprotection of the peptide derivatives.

Procedure A : The protected compound was dissolved in the smallest possible amount of a mixture of tetrahydrofuran (THF) and water in the approximate proportion 3:l. 4 equivalents of LiOH was added and the mixture was stirred until the hydrolysis was complete.

Procedure B: The procedure is as described in procedure A above but the solvent was $H_2O$:THF:DMF - (dimethylformamide) in the approximate proportion 2:l:l.

Procedure C: The deprotected compound was dissolved in THF:$H_2O$ (l:l), l0%. Pd-on-charcoal was added (20-50% of the weight of the starting material). The mixture was shaken under hydrogen (pressure 3 bar) in a Parr apparatus overnight followed by filtration and concentration.

Procedure D: The procedure is as described in procedure C but the solvent was water.

Procedure E: The procedure is as described in procedure C but the solvent was methanol.

Abbreviations used in the working examples:

Ac = acetyl
Bn = benzyl
Cbz = benzyloxycarbonyl
Me = methyl
Et = ethyl
Su = succinimido
Ph = phenyl
MeOH = methanol
EtOAC = ethyl acetate
THF = tetrahydrofuran
DCC = dicyclohexylcarbodiimide
DMF = dimethylformamide

The following references were used when recording NMR spectra:
tetramethylsilane in $CHCl_3$, $CD_3OD$ and DMF-$d_7$. In $D_2O$ tert-BuOH (δ30.6 and l.23) was used.
$R_f$ values on TLC refer to silica gel Merck 60 $F_{254}$ if nothing else is stated.

Example l Ethyl 2,3-dideoxy-4,5;7,8-di-O-isopropylidene-6-O-(trimethylsilyl)-D-manno-octonate

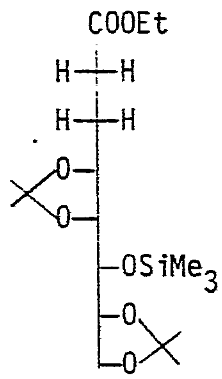

Ethyl 2,3-dideoxy-4,5;7,8-di-O-isopropylidene-D-manno-oct-2-enonate, which is a known compound - (Collins et al. Chem. Soc. Chem. Comm. l98l, ll39) is hydrogenated over l0% palladium-on-charcoal and then silylated using chlorotrimethylsilane in pyridine-ether to give after silica gel chromatography the title compound as a syrup in 90% yield.

$[\alpha]^{22}_{D}$ +38.7° ( c l.0, $CHCl_3$)

$^{13}$C-NMR ($CHCl_3$): δ172.6; 108.6; 106.9; 76.2; 75.8; 74.9; 7l.0; 66.2; 59.4; 29.9; 27.3; 25.3; 25.l; 24.5; 13.5.

Example 2 Ethyl 4,5;7,8-di-O-isopropylidene-2,3-dideoxy-2-iodo-6-O-(trimethylsilyl)-D-glycero-D-galacto-octonate

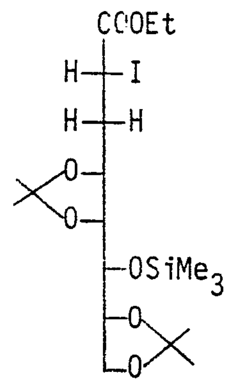

The compound from Example I (I.O g; 2.5 mmol) in IO ml of tetrahydrofuran (THF) was added during 5 min to a stirred solution of lithium diisopropylamide (2.8 mmol) in 7O ml of THF, which was kept at -78°C under nitrogen. After I5 min dry $ZnCl_2$ (O.34 g; 2.5 mmol) was added in one portion and the mixture was stirred for I hour. While keeping the temperature of the reaction mixture at -7O°C a solution of iodine (O.76 g; 3 mmol) in IO ml of THF was added. The temperature was allowed to rise to about O°C and solvents were evaporated under vacuum while the temperature was kept below 2O°C. The residue was dissolved in IOO ml of ether -light petrol (I:4) and treated with 5 ml of a saturated solution of sodium thiosulfate and sodium hydrogen carbonate in water for 5 min. The mixture was dried over $Na_2SO_4$.

Chromatography on silica gel (light petrol -ether, 3:I) gave the title compound as a syrup together with its C-2 epimer having the talo configration (O.6O and O.38 g, respectively; 74% total yield). $R_f$ values in the same solvent system were O.55 and O.44, respectively.

Physical data $[\alpha]^{22}_{D}$ 62° (c I.IO, $CHCl_3$). $^{13}C$-NMR: δI7I.O; IO9.6; I97.6; 8O.2; 77.O; 76.3; 72.O; 67.7; 6I.3; 35.7; 28.O; 26.2; 25.8; 25.I; I9.9 (CHI), I3.5 ppm.

Example 3 Ethyl 2,3-dideoxy-2-iodo-4,5;7,8-di-O-isopropylidene-D-glycero-D-talo-octonate

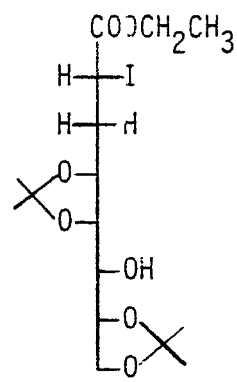

The galacto iodide from Example 2 was allowed to react with O.4 equivalents of tetrabutylammonium fluoride at room temperature in a 9:I mixture of ethyl acetate and ethanol until all starting material had disappeared (I-5 min). This treatment removed the trimethylsilyl group to give the title compound in 9O% yield after purification on silica gel (pentane/ether, I:I).

$^{13}C$-NMR ($CHCl_3$) : δI7I.I; IO9.3; IO8.2; 76.5; 76.2; 76.2; 7O.6; 67.3; 6I.6; 35.4; 27.O; 26.7; 25.2; 24.8; 2O.5; I3.6. IR: 34OO $cm^{-1}$ (strong).

Example 4 Methyl 4,5,7,8-tetra-0-acetyl-2,6-anhydro-3-deoxy-D-glycero-D-talo-octonate

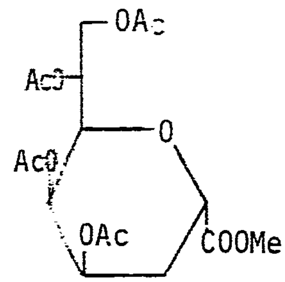

A mixture of 2.0 g (4.6 mmol) of methyl 4,5,7,8,tetra-O-acetyl-2-chloro-2,3-dideoxy-α-D-manno-2-octulosonate (Bhattacharjee et al., Biochemistry 1978, 17, 645) in 25 ml of toluene containing 1 ml of pyridine and 1 g of 10 % palladium-on-charcoal was hydrogenated in a Parr apparatus at 40 psi for 8 h. After filtration and evaporation of solvents the residue was chromatographed on silica gel with ether-pentane 2:1 as eluent to give 1.40 g (68%) of the title compound. M.p. 102-104°C.
[α] $^{22}_{D}$ + 108.8° (c 2.17, $CHCl_3$). $^{13}C$-NMR ($CHCl_3$): δ170.8-169.6 (C-1 + acetyl); 72.3 (C-2); 70.6 (C6); 68.1; 66.8; 65.0; 62.6 (C8); 52.5 (MeO); 26.5 (C3). Anal. Calc. for $C_{17}H_{24}O_{11}$:C, 50.50; H, 5.98. Found: C, 50.5; H, 6.2. From the reaction mixture was also isolated 135 mg (6%) of the C-2 epimer with galacto configuration which was briefly described by Unger et al. (Communication at the 9th International Symposium on Carbohydrate Chemistry, London 1978. Abstract No B49).

Example 5 Methyl 2,6-anhydro-3-deoxy-D-glycero-D-talo-octonate

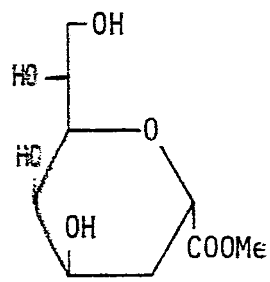

The tetraacetate-methyl ester from Example 4 was treated with 0.1 equivalents of sodium methoxide in dry methanol for 1 h at room temperature. Methanol-washed $H^+$ ion-exchange resin was added, the mixture was filtered and solvents were evaporated to give crystalline title compound (100%).

M.p. 74-75°C
[α] $^{22}_{D}$ + 76° (c 2.40, MeOH).
$^{13}C$-NMR ($D_2O$, dioxan ref.δ67.4):δ174.4; 75.6; 73.4; 70.2; 67.2; 67.0; 64.3; 53:6; 28.4.

Example 6 Ethyl 2,6-anhydro-3-deoxy-4,5;7,8-di-O-isopropylidene-D-glycero-D-talo-octonate

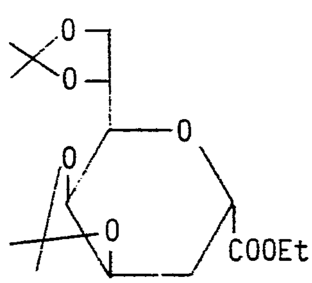

The compound from Example 3 was dissolved in ethyl acetate/ethanol (9:l) and was then treated with an excess of dry $K_2CO_3$ (about 3 equivalents). The stirred mixture was then left at room temperature for 3O h. Evaporation of solvents and silica gel chromatography (ether-pentane l:l) gave a 95% yield of the title compound.

The D-talo-iodide from Example 2 was also converted in part to the title compound by first treating it in acetonitrile with a catalytic amount of tetrabutylammonium iodide to give an equilibrium mixture of the iodides, then separating the epimers and cyclizing the isolated galacto iodide as described in the present Example.

Example 7 Ethyl 2,6-anhydro-3-deoxy-D-glycero-D-talo-octonate

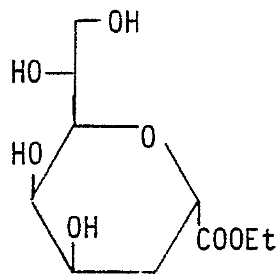

The bisacetonide (l.O8) from Example 6 was dissolved in 2O ml of methanol and 5 ml of trifluoracetic acid were added. The mixture was left for O.5 h and volatiles were then removed on a vacuum evaporator. The residue was dissolved in methanol which was removed under vacuum. This procedure was repeated three times. The residue crystallized to give O.65 g of the title compound.

M.p. l26-l28°C.

$^{13}$C-NMR (MeOH): δl73.4; 76.3; 73.8; 7l.O; 68.O; 68.O; 65.6; 62.4; 29.6; l4.5.

Example 8 Methyl 2,6-anhydro-3-deoxy-8-O-trityl-D-glycero-D-talo-octonate

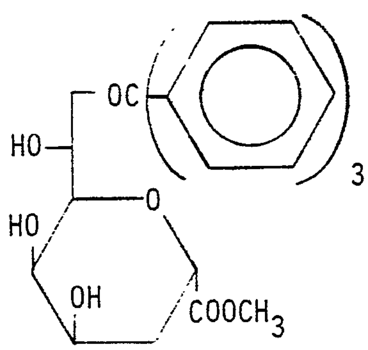

Methyl 2,6-anhydro-3-deoxy-D-glycero-D-talo-octonate (l2.3 g, 52.l mmol; Example 5) was dissolved in dry pyridine (l95 ml). Triphenylmethyl chloride (36.3 g, l3O.2 mmol) was added in portions at 3O° and the solution was stirred at that temperature overnight. Then it was poured into an ice/water mixture (75O ml) and chloroform was added (25O ml). The phases were separated and the aqueous layer was extracted with chloroform (2×llO ml). The combined chloroform phases were washed with a saturated sodium chloride solution (×2) and the pH of the aqueous layer was adjusted to 5 with sodium bisulphate. After phase separation the chloroform layer was washed with water, dried with magnesium sulphate and concentrated. Purification on silica gel with ethyl acetate/diisopropyl ether 3:2 gave the title compound (l8.6 g, 75%)

$R_f$ O.2 (ethyl acetate/diisopropyl ether 5:2).

Example 9 Methyl 2,6-anhydro-4,5,7-tri-O-benzyl-3-deoxy-8-O-trityl-D-glycero-D-talo-octonate and benzyl 2,6-anhydro-4,5,7-tri-O-benzyl-3-deoxy-8-O-trityl-D-glycero-D-talo-octonate

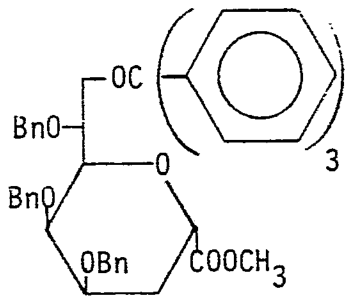
+
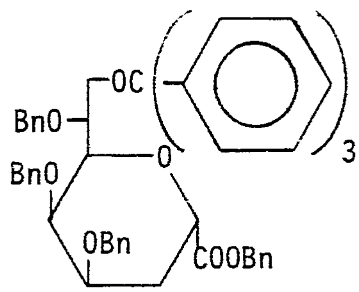

Methyl 2,6-anhydro-3-deoxy-8-O-trityl-D-glycero-D-tal-octonate (15 g, 31.4 mmol; Example 8) was dissolved in dry dimethylformamide (130 ml) and benzyl bromide (32.2 g, 188 mmol) was added. The solution was cooled to -20° and silver oxide (87.3 g, 377 mmol) was added in portions. After 30 min the temperature was raised to room temperature and the solution was stirred for 3 days in the dark. After cooling to -35° methanol (7.6 ml) in dimethylformamide (40 ml) was added dropwise and the temperature was slowly raised to room temperature. Chloroform (255 ml) was added and the reaction mixture was filtered through hyflo and then concentrated. Purification on silica gel with hexane/ethyl acetate 9:1 gave:

a: methyl 2,6-anhydro-4,5,7-tri-O-benzyl-3-deoxy-8-O-trityl-D-glycero-D-talo-octonate (0.8 g, 3%)

$R_f$ 0.32 (hexane/diisopropyl ether 1:1)

$[\alpha]_D^{20}$ + 15.2 (c 1.02, chloroform)

$^{13}$C-NMR ($CDCl_3$): δ172.1 (C1); 144.2 (C, arom.); 126.7-128.9 (CH, arom.); 86.6 (C, trityl); 76.7; 76.1; 74.5; 74.1 - ($CH_2$); 72.8; 72.1 ($CH_2$); 70.4 ($CH_2$); 63.1 (C8); 51.7 ($OCH_3$); 27.0 (C3).

b: Benzyl 2,6-anhydro-4,5,7-tri-O-benzyl-3-deoxy-8-O-trityl-D-glycero-D-talo-octonate (0.45 g, 2%)

$R_f$ 0.42 (hexane/diisopropyl ether 1:1)

$^{13}$C-NMR ($CDCl_3$): δ171.4 (C1); 144.3; 139.3; 138.7; 138.4; 135.7; 133.6; 130.2 (C, arom.); 126.8-129.0 (CH, arom.); 86.8 (C, trityl); 76.4; 74.7; 74.1 ($CH_2$); 72.9; 72.8 ($CH_2$); 72.4; 70.5 ($CH_2$); 66.5 ($CH_2$); 64.0 (C8); 27.2 (C3).

c: A mixture of methyl 2,6-anhydro-4,5,7-tri-O-benzyl-3-deoxy-8-O-trityl-D-glycero-D-talo-octonate and benzyl 2,6-anhydro-4,5,7-tri-O-benzyl-3-deoxy-8-O-trityl-D-glycero-D-talo-octonate (6.7 g, 27%).

Example 10 Methyl 2,6-anhydro-4,5,7-tri-O-benzyl-3-deoxy-D-glycero-D-talo-octonate and benzyl 2,6-anhydro-4,5,7-tri-O-benzyl-3-deoxy-D-glycero-D-talo-octonate

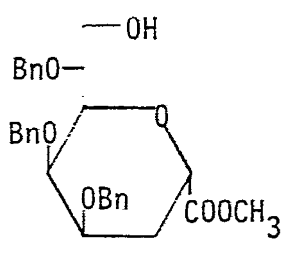
+
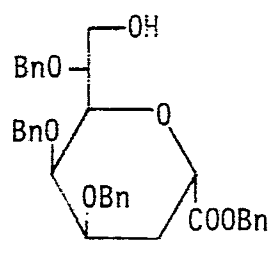

The mixture of methyl 2,6-anhydro-4,5,7-tri-O-benzyl-3-deoxy-8-O-trityl-D-glycero-D-talo-octonate and benzyl 2,6-anhydro-4,5,7-tri-O-benzyl-3-deoxy-8-O-trityl-D-glycero-D-talo-octonate (6.7 g, 9 mmol; Example 9) was detritylated in methanol/toluene (740 ml; 10:1) containing IR 120 ($H^+$) (76 ml). The reaction mixture was heated at 70° overnight and after filtration and concentration the residue was purified on silica gel with hexane/ethyl acetate 2:1. Chromatography gave

a: methyl 2,6-anhydro-4,5,7-tri-O-benzyl-3-deoxy-D-glycero-D-talo-octonate (2.47 g, 54%)

$R_f$ 0.18 (hexane/ethyl acetate 3:2)

$[\alpha]_D^{20}$ + 23.6 (c 1.43, chloroform)

$^{13}$C-NMR ($CDCl_3$): δ172.6 (C1); 139.0; 138.3; 138.2 (C, arom.); 127.5-129.1 (CH, arom.); 77.0; 76.2; 74.7; 74.2 - ($CH_2$); 73.0; 72.3; 71.3 ($CH_2$); 70.5 ($CH_2$); 60.5 (C8); 52.5 ($OCH_3$); 27.0 (C3).

b: Benzyl 2,6-anhydro-4,5,7-tri-O-benzyl-3-deoxy-D-glycero-D-talo-octonate (l.5 g, 29%)
$R_f$ 0.29 (ethyl acetate/hexane 3:2)
$[\alpha]_D^{20}$ + 8.6 (C l.l7, chloroform)
$^{13}$C-NMR ($CDCl_3$): δl7l.9 (Cl); l38.9; l38.7; l38.2; l35.2 (C. arom.); l27.l-l28.7 (CH, arom.); 76.9; 76.2; 74.3; 74.l ($CH_2$); 72.8; 72,2; 7l.2 ($CH_2$); 70.4 ($CH_2$); 67.3 ($CH_2$); 60.3 ($CH_2$); 27.0 (C3).

Example ll Benzyl 2,6-anhydro-3-deoxy-D-glycero-D-talo-octonate

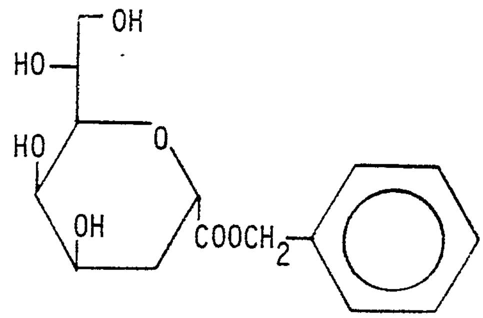

Methyl 2,6-anhydro-3-deoxy-D-glycero-D-talo-octonate (8.l g, 34.3 mmol; Example 5) was transesterified in benzyl alcohol (l70 ml) and l.5 M n-butyllithium (3.0 ml) under dry conditions overnight. Neutralization with Amberlite IR l20 ($H^+$) and continuous concentrations with water gave the title compound (9.2 g, 86%).
$R_f$ 0.32 (ethyl acetate/methanol/toluene 7:2:l)
$^{13}$C-NMR ($CD_3OD$): δl73.3 (Cl); l37.2 (C, arom.); l29.7; l29.5 (CH, arom.); 76.5; 73.9; 7l.2; 68.l; 68.0; 65.7 -(C8); 29.7 (C3).

Example l2 Benzyl 2,6-anhydro-3-deoxy-8-O-trityl-D-glycero-D-talo-octonate

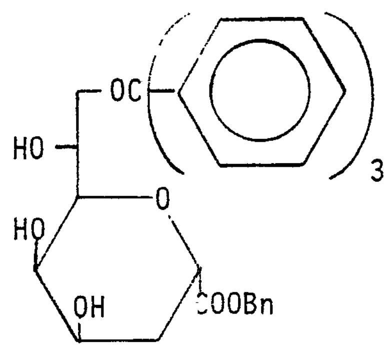

Benzyl 2,6-anhydro-3-deoxy-D-glycero-D-talo-octonate (l.94 g, 6.2 mmol; Example ll) was tritylated with triphenylmethyl chloride (4.3 g, l5.5 mmol) in dry pyridine (20 ml) as in Example 8. Purification with ethyl acetate/hexane l:l, ethyl acetate/hexane 3:l and ethyl acetate gave the title compound (l.9 g, 55%).
$R_f$ 0.44 (ethyl acetate)
$^{13}$C-NMR ($CDCl_3$): δl7l.2 (Cl); l43.8 (C, arom.); l27.l-l28.7 (CH, arom.); 87.l (C, trityl); 74.0; 72.3; 70.6-.4; 66.8; 66.3; 64.6; 29.3 (C3).

Example l3 Benzyl 2,6-anhydro-4,5,7-tri-O-benzyl-3-deoxy-8-O-trityl-D-glycero-D-talo-octonate.

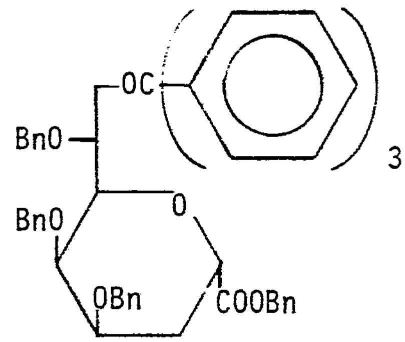

Benzyl 2,6-anhydro-3-deoxy-8-O-trityl-D-glycero-D-talo-octonate (l.7 g, 3.l mmol; Example l2) was benzylated according to the same procedure as in Example l9. This gave the title compound (0.52 g, 20%).

$R_f$ and NMR as in Example 9.

Example l4 Benzyl 2,6-anhydro-8-azido-4,5,7-tri-O-benzyl-3,8-dideoxy-D-glycero-D-talo-octonate

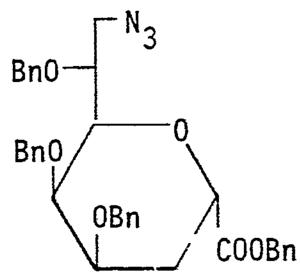

Triphenylphosphine (0.42 g, l.6 mmol) was added to a solution of benzyl 2,6-anhydro-4,5,7-tri-O-benzyl-3-deoxy-D-glycero-D-talo-octonate (0.62 g, l.l mmol; Example l0), lithium azide (0.37 g, 7.6 mmol) and tetrabromomethane (0.53 g, l.6 mmol) in dimethylformamide (l0 ml) at 0° under dry conditions and the mixture was stirred at room temperature overnight. The solvent was evaporated, diethyl ether was added to the residue and the salts were filtered off. Concentration and purification on silica gel with ethyl acetate/toluene l:30 gave the title compound (0.49 g, 76%). Recrystallization took place from diethyl ether/petroleum ether.

M.p. 50-5l°

$R_f$0.58 (diisopropyl ether/hexane 4:l)

$[\alpha]_D^{22}$ +l6 (c l.6, chloroform)

IR:$\nu$ $CHCl_3$ 20l0 $cm^{-1}$ (azide), l730 $cm^{-1}$

$^1$H-NMR ($CDCl_3$): $\delta$2.30-2.5l (m, 2 H-3); 3.34-4.68 (m); 5.03-5.25 (m, $CH_2$, benzyl); 7.24-7.36 (m, arom.)

$^{13}$C-NMR ($CDCl_3$): $\delta$l7l.2 (Cl); l38.9; l38.2; l37.8; l35.5 (C, arom.); l27.3-l28.6 (CH, arom.); 76.5; 76.3; 74.6; 74.l ($CH_2$); 72.9; 72.6; 72.4 ($CH_2$); 70.5 ($CH_2$); 66.9 ($CH_2$); 5l.l (C8); 27.l (C3).

Example l5 Benzyl 2,6-anhydro-8-azido-3,8-dideoxy-D-glycero-D-talo-octonate

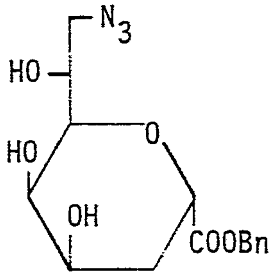

Tetrabromomethane (973 mg, 2.9 mmol) and triphenylphosphine (769 mg, 2.9 mmol) were added to a solution of benzyl 2,6-anhydro-3-deoxy-D-glycero-D-talo-octonate (458 mg, l.46 mmol; Example ll) and lithium azide (5O3 mg, lO.3 mmol) in dimethylformamide (ll ml) under dry conditions. After stirring overnight an additional amount of tetrabromomethane (5OO mg, l.5 mmol) and triphenylphosphine (4OO mg, l.5 mmol) was added. Concentration and purification on silica gel with chloroform/methanol l2:l gave the title compound (27O mg, 54%).

$R_f$ O.5 (chloroform/methanol 8:l)

$^{13}$C-NMR ($CD_3OD$): δl72.8 (Cl); l37 (C, arom.); l29.6; l29.5 (CH, arom.); 76.8; 74.O; 69.9; 68.O; 67.9 ($CH_2$, benzyl); 67.8; 55.9 (C8); 29.6 (C3).

Example l6 Methyl 2,6-anhydro-4,5,7-tri-O-benzyl-8-O-tosyl-3-deoxy-D-glycero-D-talo-octonate

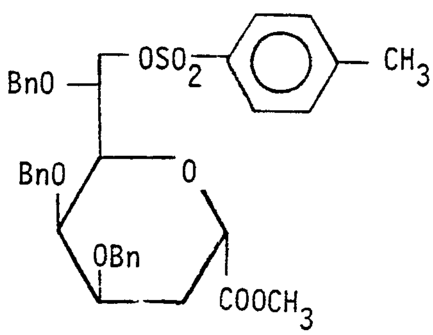

Tosylation of methyl 2,6-anhydro-4,5,7-tri-O-benzyl-3-deoxy-D-glycero-D-talo-octonate (l.l g, 2.l mmol; Example lO) was carried out with p-toluenesulphonyl chloride (8OO mg, 4.2 mmol) in acetone (O.7 ml) and pyridine (O.4 ml) at O°, the mixture being stirred at room temperature overnight. The reaction mixture was concentrated and the residue was dissolved in dichloromethane. Purification on silica gel with toluene and toluene/ethyl acetate 2:l afforded methyl 2,6-anhydro-8-O-tosyl--4,5,7-tri-O-benzyl-3-deoxy-D-glycero-D-talo-octonate (l.3 g, 93%).

$R_f$ O.3 (diisopropyl ether/hexane 4:l)

$^{13}$C-NMR ($CDCl_3$): δl7l.9 (Cl); l44.5; l38.9; l38, l; l37.7 (C, arom); l27.4-l29.7 (CH, arom.); 75.9; 75.O; 74.l; 73,8; 72.9; 72.4; 72.2; 7O.4; 69.2; 52.l ($OCH_3$); 26.9 (C3); 2l.5 ($CH_3$, arom.).

Example l7 Methyl 2,6-anhydro-8-azido-3,8-dideoxy-D-glycero-D-talo-octonate

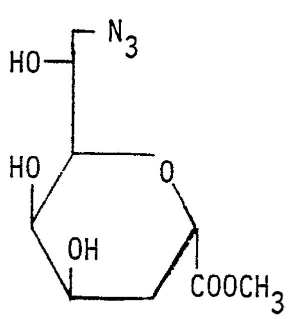

Methyl 2,6-anhydro-3-deoxy-D-glycero-D-talo-octonate (275 mg, 1.16 mmol; Example 5) and lithium azide (400 mg, 8.15 mmol) were dissolved in dimethylformamide (6 ml) under a nitrogen atmosphere. Tetrabromomethane (915 mg, 3.5 mmol) and triphenylphosphine (915 mg, 3.5 mmol) were added and the reaction was stirred for 15 minutes at 0° and for 3 days at room temperature. Methanol (1 ml) was then added and the reaction mixture was concentrated after 30 minutes. Purification on silica gel with chloroform/methanol 12:1 gave the title compound (212 mg, 70%).

$R_f$ 0.41 (chloroform/methanol 8:1)

$^{13}C$-NMR ($CD_3OD$): δ173.6 (C1); 76.5; 73.8; 69.8; 67.7; 55.6 (C8); 52.7 ($OCH_3$); 29.4 (C3).

Example 18 2,6-anhydro-8-azido-3,8-dideoxy-D-glycero-D-talo-octonic acid

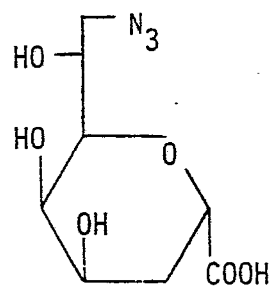

Methyl 2,6-anhydro-8-azido-3,8-dideoxy-D-glycero-D-talo-octonate (99 mg, 0.38 mmol; Example 17) was hydrolyzed in 1 M sodium hydroxide (1 ml) for 1 hour. Conversion to the acid with DOWEX 50 W x 8 ($H^+$) ion-exchange resin afforded after concentration the title compound (82 mg, 88%).

$^1H$-NMR ($D_2O$): δ1.98-2.11 (m, 2H, H-3); 3.36-3.94 (m, 6H); 4.58 (dd, 1H).

Example 19 Benzyl 2,6-anhydro-8-azido-4,5,7-tri-O-benzyl-3,8-dideoxy-D-glycero-D-talo-octonate

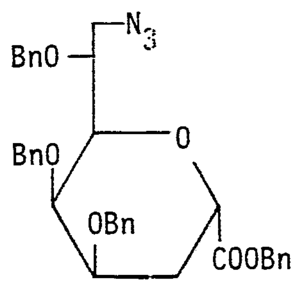

2,6-Anhydro-8-azido-3,8-dideoxy-D-glycero-D-talo-octonic acid (80 mg, 0.32 mmol; Example 18) was dissolved in 1,2-dimethoxyethane/dimethyl formamide 1:1 (1.6 ml). Benzyl bromide (0.46 ml, 3.9 mmol) and tetrabutylammonium iodide (0.28 g, 0.8 mmol) were added followed by addition of sodium hydride (85 mg, 1.9 mmol; 60% in liquid paraffin) at 0°. The mixture was stirred at room temperature for 40 hours. After addition of ammonium chloride (0.1 g, 1.9 mmol) and triethylamine (0.54 ml, 3.9 mmol) the reaction mixture was concentrated. Diethyl ether was added to the residue and the salts were filtered off. Concentration and purification on silica gel with ethyl acetate/toluene 1:50 afforded the title compound (80 mg, 40%).

$R_f$, IR and NMR as in Example 14.

Example 20 Benzyl 8-amino-2,6-anhydro-4,5,7-tri-O-benzyl-3,8-dideoxy-D-glycero-D-talo-octonate

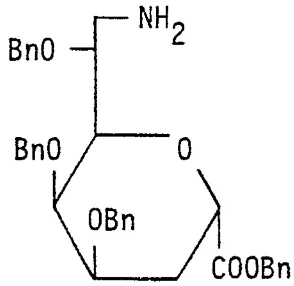

Triphenylphosphine (1.56 g, 5.9 mmol) and benzyl alcohol (2 ml) in tetrahydrofuran (6 ml) were added to a solution of benzyl 2,6-anhydro-8-azido-4,5,7-tri-O-benzyl-3,8-dideoxy-D-glycero-D-talo-octonate (520 mg, 0.85 mmol; Example 14 or 19) in tetrahydrofuran (2 ml) under dry conditions. After stirring overnight, concentration followed by purification on silica gel with chloroform/toluene 1:1, chloroform/toluene/methanol 10:10:0.5 and chloroform/toluene/methanol 10:10:1 gave the title compound (424 mg, 85%).

$R_f$ 0.37 (Chloroform/toluene/methanol 5:2:1).

$^{13}C$-NMR ($CDCl_3$): δ171.7 (C1); 139; 138.3; 130 (C, arom.); 127.3-128.7 (CH, arom.); 76.5; 74.2; 73.8; 72.4; 72.6; 71.1; 70.5; 67.1; 39.9 (C8); 27.2 (C3).

Example 21 Benzyl N-(Cbz-L-alanyl)-8-amino-2,6-anhydro-4,5,7-tri-O-benzyl-3,8-dideoxy-D-glycero-D-talo-octonate

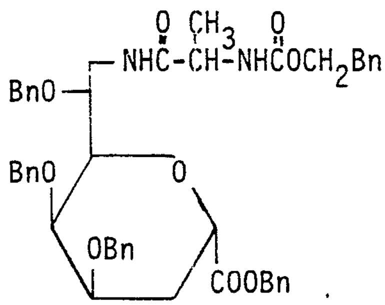

Cbz-L-alanin (65 mg, 0.3 mmol), 1-hydroxybenzotriazole (23 mg, 0.17 mmol) and triethylamine (30 mg, 0.3 mmol) were added to a solution of benzyl 8-amino-2,6-anhydro-4,5,7-tri-O-benzyl-3,8-dideoxy-D-glycero-D-talo-octonate (85 mg, 0.15 mmol; Example 20) in dichloromethane (3.5 ml). Dicyclohexylcarbodiimide (DCC) (35 mg, 0.17 mmol) was then added at 0° and the reaction was stirred at room temperature overnight. Diethyl ether (5 ml) was added and dicyclohexyl urea was filtered off. Concentration and purification on silica gel with ethyl acetate/hexane 1:2 gave after crystallization from diethyl ether/petroleum ether the title compound (63 mg, 53%).

$R_f$ 0.17 (Ethyl acetate/hexane 2:3)

$^{13}C$-NMR ($CDCl_3$):δ172.8; 172.5; 155.5 (C=O); 138.6; 138.0; 137.7; 136.7; 135.0 (C, arom); 127.3-128.8 (CH, arom.); 76.1; 74.8; 74.6; 74.3 ($CH_2$); 72.7: 71.8; 70.7 ($CH_2$); 70.5 ($CH_2$; 67.6 ($CH_2$); 66.6 ($CH_2$); 50.8 (CH, L-ala); 36.5 (C8); 27.1 (C3); 20.0 ($CH_3$, L-ala).

Example 22 Benzyl N-(Cbz-L-alanyl-L-alanyl)-8-amino-2,6-anhydro-4,5,7-tri-O-benzyl-3,8-dideoxy-D-glycero-D-talo-octonate

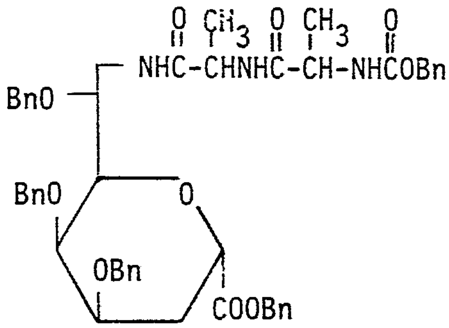

The reaction between benzyl 8-amino-2,6-anhydro-4,5,7-tri-O-benzyl-3,8-dideoxy-D-glycero-D-talo-octonate (212 mg, 0.36 mmol; Example 20), Cbz-L-alanyl-L-alanine (212 mg; 0.72 mmol), 1-hydroxybenzotriazole (54 mg, 0.40 mmol), triethylamine (73 mg, 0.72 mmol) and dicyclohexylcarbodiimide (83 mg, 0.40 mmol) in dichloromethane (10 ml) was performed by the same procedure as in Example 21. Chromatography on silica gel with ethyl acetate/hexane 1:2 gave the title compound (230 mg, 74%). The compound was recrystallized from warm ethyl acetate.

M.p. 141-142°C.

$R_f$ 0.16 (ethyl acetate/hexane 1:1)

$[\alpha]_D^{20}$ + 7.9° (c 1.2, chloroform)

$^{13}$C-NMR ($CDCl_3$): δ172.6; 172.5, 171.2, 155.7 (carbonyles) 138.6; 138.0; 137.7; 136.4; 135.0 (C, arom.); 127.3-128.8 (CH, arom.); 76.1; 74.8; 74.6; 74.3 ($CH_2$); 72.8; 71.8; 70.7 ($CH_2$); 70.5 ($CH_2$); 67.7 ($CH_2$); 66.8 ($CH_2$); 50.6, 49.1 (CH, alanyl); 36.6 (C8); 27.1 (C3); 19.6; 19.1 ($CH_3$, alanyl).

Example 23 Methyl 2,6-anhydro-4,5,7-tri-O-benzyl-3,8-dideoxy-8-fluoro-D-glycero-D-talo-octonate

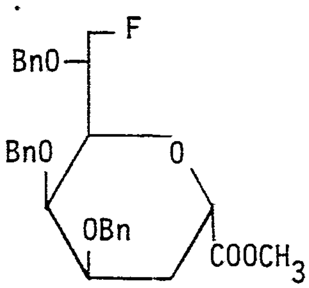

A solution of methyl 2,6-anhydro-4,5,7-tri-O-benzyl-3-deoxy-D-glycero-D-talo-octonate (635 mg, 0.8 mmol; Example 10) and triethylamine (0.12 ml, 0.88 mmol) in dichloromethane (2.4 ml) was added dropwise to a solution of diethylaminosulfur trifluoride (0.15 ml, 1.2 mmol) in dichloromethane (2.4 ml) at -40° under a nitrogen atmosphere. The temperature was slowly raised to room temperature. After one hour the reaction mixture was diluted with dichloromethane (20 ml) and 6% aqueous sodium bicarbonate (40 ml) was added dropwise. The layers were separated and the aqueous layer was extracted with dichloromethane (×2). The combined phase extracts were washed with water (×3) and dried (magnesium sulfate). Chromatography on silica gel with hexane/ethyl acetate 5:1 gave the title compound (187 mg, 29%).

M.p. 60-62°C

$R_f$ 0.52 (hexane/ethyl acetate 3:2)

$^{13}$C-NMR ($CDCl_3$)δ172.0 (C1); 138.9; 138.1 (C, arom.); 127.4-128.4 (CH, arom.); 83.6 (d, C8, $^1J_{C-F}$211); 76.0; 74.2; 73.7; 73.5; 72.9; 72.4; 70.5; 52.1 ($OCH_3$); 27.0 (C3).

Example 24 Methyl 2,6-anhydro-3,8-dideoxy-8-fluoro-D-glycero-D-talo-octonate

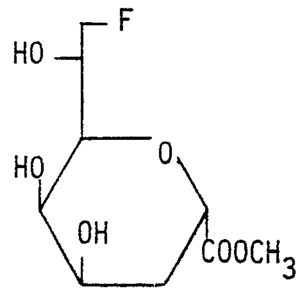

Methyl 2,6-anhydro-4,5,7,tri-O-benzyl-3,8-dideoxy-8-fluoro-D-glycero-D-talo-octonate (193 mg, 0.38 mmol; Example 33) was debenzylated by hydrogenation in methanol/tetrahydrofuran (10 ml, 3:1) with 5% palladium on charcoal (35 mg). An additional amount of the catalyst (35 mg) was added after 6 hours and after another 24 hours the catalyst was filtered off. Purification on silica gel with ethyl acetate/hexane 3:2 and ethyl acetate/methanol/toluene, 7:2:1 gave the title compound (61 mg, 68%).

$R_f$ 0.41 (ethyl acetate/methanol/toluene 7:2:1)

$[\alpha]^{20}_{D}$ +73.8° (c 0.52, methanol)

$^{13}$C-NMR ($CD_3OD$): δ173.7 (C1); 86.4 (d, C8 $^1J_{C-F}$ 167); 75.4 (d, C6 $^3J_{C-F}$7); 74.0; 69.8 (d, C7, $^2J_{C-F}$18); 68.0; 52.7 ($OCH_3$); 29.8 (C3).

Example 25 Methyl 2,6-anhydro-4,5,7-tri-O-benzyl-3,8-dideoxy-8-iodo-D-glycero-D-talo-octonate

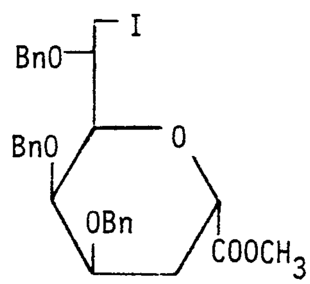

Methyl 2,6-anhydro-4,5,7-tri-O-benzyl-3-deoxy-8-O-toxyl-D-glycero-D-talo-octonate (200 mg, 0.3 mmol; Example 16) was dissolved in dry dimethylformamide (0.6 ml) and sodium iodide (114 mg, 0.76 mmol) was added. The solution was heated at 85° for 2.5 hours and after cooling it was added dropwise to a mixture of cold water (15 ml) and ethyl acetate (15 ml). After separation the water was extracted with ethyl acetate (15 ml) and the combined phase extracts were washed with 5% sodium thiosulfate (15 ml) and water (2×7 ml). Drying with sodium sulfate, filtration and concentration gave the title compound.

$R_f$ 0.43 (diisopropyl ether/hexane 4:1)

$[\alpha]^{20}_{D}$ -6° (c 1.5, chloroform)

$^{13}$C-NMR ($CDCl_3$): δ171.6 (C1); 139.1; 138.3; 137.5 (C, arom.); 127.4-128.4 (CH, arom.); 76.4; 75.9; 74.3; 74.0; 73.1; 72.6; 71.1; 70.5; 52.2 ($OCH_3$); 26.9 (C3); 9.5 (C8).

Example 26 Methyl 2,6-anhydro-4,5,7-tri-O-benzyl-3,8-dideoxy-D-glycero-D-talo-octonate

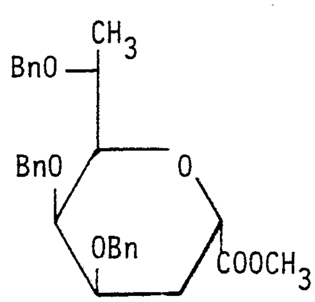

Reduction of methyl 2,6-anhydro-4,5,7-tri-O-benzyl-3,8-dideoxy-8-iodo-D-glycero-D-talo-octonate (138 mg, 0.34 mmol; Example 25) was performed by hydrogenation overnight in methanol/tetrahydrofuran (4 ml, 3:1) and triethylamine (26 mg, 0.26 mmol) with 5% palladium on charcoal (65 mg) as catalyst. An additional amount of the catalyst (70 mg) was added and the hydrogenation continued for another 24 hours. The catalyst was filtered off and the solvent was evaporated. The residue was dissolved in ethyl acetate which was washed with water, 1 N hydrochloric acid and water. Drying and concentration gave the title compound (106 mg, 100%).

$R_f$ 0.37 (diisopropyl ether/hexane 4:1).

Example 27 Methyl 2,6-anhydro-3,8-dideoxy-D-glycero-D-talo-octonate

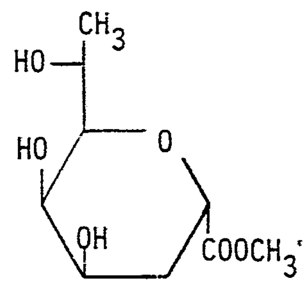

Methyl 2,6-anhydro-4,5,7-tri-O-benzyl-3,8-dideoxy-D-glycero-D-talo-octonate (138 mg, 0.28 mmol; Example 26) was debenzylated by hydrogenation overnight in methanol/tetrahydrofuran (4 ml 3:1) with 5% palladium on charcoal (65 mg) as catalyst. The catalyst was filtered off and the solvent was evaporated. Purification on silica gel with ethyl acetate/methanol/toluene 7:2:1 gave the title compound.

$R_f$ 0.21 (ethyl acetate/methanol/toluene 7:2:1)

$[\alpha]_D^{20}$ + 73° (c 0.83, methanol)

$^{13}$C-NMR ($CD_3OD$): δ174.0 (C1); 80.3; 74.1; 68.5; 68.3; 67.1; 52.7; ($OCH_3$); 30.0 (C3); 20.9 (C8).

Example 28 Methyl 8-S-acetyl-2,6-anhydro-3,8-dideoxy-8-thio-D-glycero-D-talo-octonate

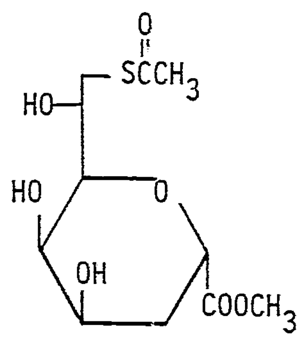

Triphenylphosphine (550 mg, 2.1 mmol) was dissolved in tetrahydrofuran (5 ml). At 0° diethyl azodicarboxylate (325 µl, 2.1 mmol) was added and when the phosphonium salt solidified a solution of methyl 2,6-anhydro-3-deoxy-D-glycero-D-talo-octonate (249 mg, 1.05 mmol; Example 5) in tetrahydrofuran - (3 ml) and acetonitrile (2 ml) was added. Stirring at 0° for 10 minutes and at room temperature for 1 hour gave a clear solution. Concentration and purification on silica gel with dichloromethane/methanol 19:1 gave the title compound (170 mg, 55%).

$R_f$ 0.57 (ethyl acetate/methanol/toluene 7:2:1)

$^{13}$C-NMR ($CDCl_3$): δ198 (SAc); 171.9; 76.0; 72.5; 69.2; 66.9; 66.6; 52.1 ($OCH_3$); 33.5 (C8); 30.5(Ac); 28.8 - (C3).

Example 29 Methyl 2,6-anhydro-3,8-dideoxy-8-thio-D-glycero-D-talo-octonate

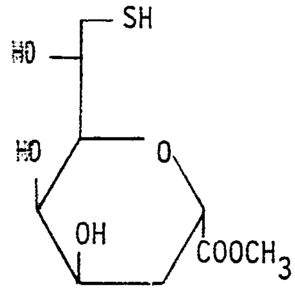

Methyl 8-S-acetyl-2,6-anhydro-3,8-dideoxy-8-thio-D-glycero-D-talo-octonate (65 mg, 0.22 mmol; Example 28) was deacetylated with sodium methoxide (1-2 eqs) in methanol under dry and oxygen-free conditions for 20 minutes. Neutralization with Amberlite IR 120 ($H^+$) was followed by filtration and concentration. Chromatography on silica gel with ethyl acetate/toluene/methanol 8:1:1 gave the title compound (35 mg, 67%)

$R_f$ 0.38 (ethyl acetate/methanol/toluene 8:1:1)

$^{13}$C-NMR ($CD_3OD$): δ173.9 (C1); 77.4; 74.1; 70.9; 68.4; 68.2; 52.8 ($OCH_3$); 29.8 (CB); 29.8 (C3).

Example 30 Ammonium 2,6-anhydro-3,8-dideoxy-8-thio-D-glycero-D-talo-octonate

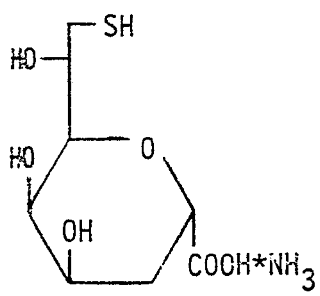

Methyl 2,6-anhydro-3,8-dideoxy-8-thio-D-glycero-D-talo-octonate (35 mg, 0.14 mmol; Example 29) was hydrolyzed in 0.5 M sodium hydroxide (0.8 ml) for 30 minutes under nitrogen. Neutralization with Amberlite IR 120 ($H^+$), and filtration followed by addition of ammonium hydroxide to pH 8 and concentration gave the title compound (35 mg, 100%).

$R_f$ 0.5 (chloroform/methanol/water 5:5:1)

$^{13}$C-NMR ($D_2O$):δ-178.9 (C1); 75.6; 75.1; 70.2; 67.2; 29.4; ($CH_2$); 29.0 ($CH_2$).

Example 31 Ammonium 2,6-anhydro-3,8-dideoxy-D-glycero-D-talo-octonate

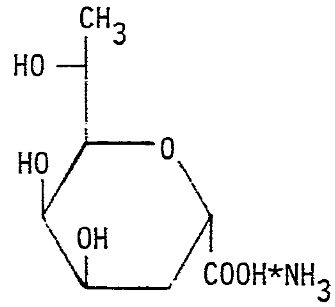

Methyl 2,6-anhydro-3,8-dideoxy-D-glycero-D-talo-octonate (16 mg, 0.07 mmol; Example 27) was hydrolyzed in 0.5 M sodium hydroxide (2 ml) for 20 minutes. The solution was then passed through an ammonium saturated cation exchange resin (Dowex 50X*8) with water as eluent. The eluate was concentrated and gave the title compound (15 mg, 100%).

$[\alpha]^{20}_{D}$ + 57° (c 0.7, methanol)

$^{13}$C-NMR ($D_2O$ δ179.1 (C1); 78.3; 75.1; 67.8; 67.1; 29.6 (C3); 20.2 (C8).

Example 32 Ammonium 2,6-anhydro-3,8-dideoxy-8-fluoro-D-glycero-D-talo-octonate

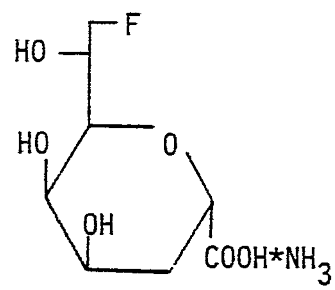

Methyl 2,6-anhydro-3,8-dideoxy-8-fluoro-D-glycero-D-talo-octonate (59 mg, 0.25 mmol; Example 24) was hydrolyzed in 0.5 M sodium hydroxide (4.5 ml) for 20 minutes. The solution was then passed through an ammonium saturated cation exchange resin (Dowex 50Wx8) with water as eluent. The eluate was concentrated and the residue was recrystallized from water/ethanol to give the title compound (52 mg, 87%).

M.p. 147-149°. $[\alpha]^{20}_{D}$ + 66° (c 1.06, water)

$^{13}$C-NMR ($D_2O$): δ178.5 (C1); 86.2 (d, C8, $^1J_{C-F}$ 165); 74.9; 75.3 (d, C67, $^3J_{C-F}$7); 69.2 (d, C7, $^2J_{C-F}$18)); 67.5; 67.5; 29.4 (C3).

Example 33 Ammonium 8-azido-2,6-anhydro-3,8-dideoxy-D-glycero-D-talo-octonate

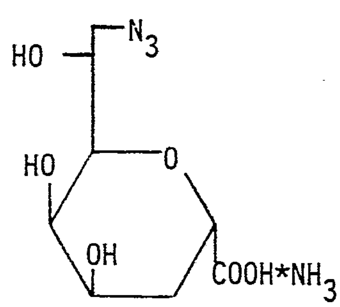

Methyl 2,6-anhydro-8-azido-3,8-dideoxy-D-glycero-D-talo-octonate (lOO mg, 0.38 mmol; Example l7) was hydrolyzed in 0.5 M sodium hydroxide (3 ml) for 40 minutes. The solution was passed through an ammonium saturated cation exchange resin with water as eluent and the eluate was freeze-dried. The title compound (92 mg, 90%) was obtained as a white powder.

$^{13}C$-NMR ($D_2O$):δ178.7 (Cl); 75.2; 75.0; 69.6; 67.6; 54.9 (C8); 29.4 (C3).

Example 34 8-Amino-2,6-anhydro-3,8-dideoxy-D-glycero-D-talo-octonic acid

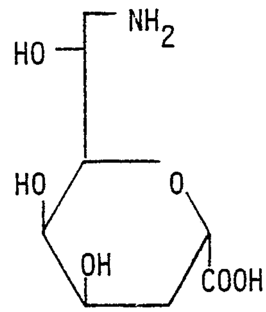

Benzyl 2,6-anhydro-8-azido-3,8-dideoxy-D-glycero-D-talo-octonate (0.5 g, l.48 mmol; Example l5) was reduced and deprotected by catalytic hydrogenation in tetrahydrofuran/water (l6 ml, 5.3) with l0% palladium on charcoal (l75 mg) for 2 hours. The catalyst was filtered off and after concentration and dissolution in water the product was purified by ion exchange chromatography (Dowex 50W x 8[$H^+$]) with water and then ammonium hydroxide as eluents. The eluate from ammonia was concentrated and crystallisation from water gave the title compound (l52 mg, 46%).

M.p.: decomposition above 300°C

$R_f$ 0.45 (propanol/water 3:2)

$^{13}C$-NMR ($D_2O$): δ179.3 (Cl); 75.3; 75.0; 67.6; 66.8; 65.4; 44.2 (C8); 29.2 (C3).

Example 35 8-Amino-2,6-anhydro-3,8-dideoxy-D-glycero-D-talo-octonic acid

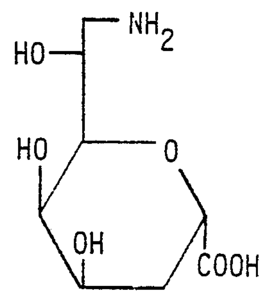

Benzyl 2,6-anhydro-8-azido-4,5,7-tri-O-benzyl-3,8-dideoxy-D-glycero-D-talo-octonate (0.5 g, 0.68 mmol; Example l4 or l9) was deprotected by catalytic hydrogenation overnight in tetrahydrofuran/water/ethanol l:2:7 with 5% palladium on charcoal (520 mg) as catalyst. The catalyst was filtered off and after concentration the residue was purified on silica gel with propanol/water 7:3. Crystallization from water/ethanol gave the title compound (60 mg, 40%).

M.P., $^1H$-NMR and $^{13}C$-NMR as in Example 34.

Example 36Ethyl 8-amino-2,6-anhydro-3,8-dideoxy-D-glycero-D-talo-octonate hydrochloride

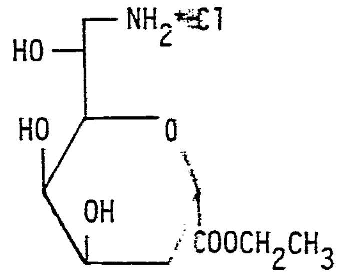

Hydrogenolysis of methyl 2,6-anhydro-8-azido-3,8-dideoxy-D-glycero-D-talo-octonate (I7O mg, O.65 mmol; Example I7) was performed in methanol (5 ml) with IO% palladium on charcoal (II5 mg) as catalyst. Ion exchange chromatography on DOWEX 5OW×8 ($H^+$) (prewashed with methanol) with methanol and then IM-hydrochloric acid as eluent afforded 8-amino-2,6-anhydro-3,8-dideoxy-D-glycero-D-talo-octonic acid (IIO mg) which was esterified with thionyl chloride (75 mg, O.63 mmol) in absolute ethanol to give the title compound (45 mg, 28%) after chromatography on silica gel with propanol/water 3:I.

$^{13}$C-NMR ($D_2O$): δI74.O (CI); 77.I; 73.5; 66.8; 66.I; 63.7; 43.8 (C8); 28.3 ($C_3$); 14.2 ($CH_3$)

Example 37 N-(L-alanyl)-8-amino-2,6-anhydro-3,8-dideoxy-D-glycero-D-talo-octonic acid

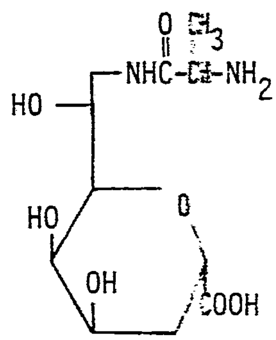

Benzyl N-(Cbz-L-alanyl)-8-amino-2,6-anhydro-4,5,7-tri-O-benzyl-3,8-dideoxy-D-glycero-D-talo-octonate (63 mg, O.O8 mmol; Example 2I) was hydrogenated at 4 atm for I6 hours in tetrahydrofuran/water (3 ml, 3:I) with IO% palladium on charcoal (2OO mg) as catalyst. The catalyst was filtered off and washed with water. After neutralization with IM ammonium hydroxide the filtrate was concentrated. Continuous concentrations with ethanol gave the title compound (23 mg, IOO%).

$R_f$ O.45 (propanol/water 3:2)

$^{13}$C-NMR ($D_2O$): δI79.O (CI); I72.I (amide); 75.4; 75.2; 68.O; 67.7; 67.O; 5O.2; (CH, L-ala); 43.5 (C8); 29.4 (C3); I7.6 ($CH_3$).

Example 38 N-(L-alanyl-L-alanyl)-8-amino-2,6-anhydro-3,8-dideoxy-D-glycero-D-talo-octonic acid

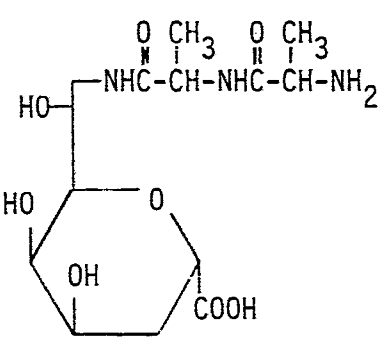

Benzyl N-(Cbz-L-alanyl-L-alanyl)-8-amino-2,6-anhydro-4,5,7-tri-O-benzyl-3,8-dideoxy-D-glycero-D-talo-octonate (2lO mg, O.24 mmol; Example 22) was hydrogenated by the same procedure as in Example 37 with O.5 g catalyst in tetrahydrofuran/water 3:l. Concentration after neutralization follwed by precipitation from methanol/ether gave the title compound (6O mg, 69%).

$R_f$ O.39 (propanol/water 3:2)

$^{13}$C-NMR ($D_2O$): δl79.O; l75.6; l7l;l (carbonyls); 75.2; 75.O; 68.2; 67.6; 67.l; 5O.9; 49.8; (CH, L-ala); 43.3 - (C8); 29.4 (C3); l7.7 ($CH_3$); l7.l ($CH_3$).

Example 39 Methyl 8-amino-2,6-anhydro-3,8-dideoxy-D-glycero-D-talo-octonate

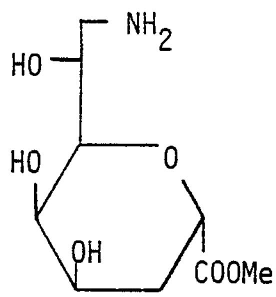

Methyl 2,6-anhydro-8-azido-3,8-didexoy-D-glycero-D-talo-octonate (2 g, 7.65 mmol; Example l7) in MeOH/THF/EtOAc l:2:8 (55 ml) was reduced by catalytical hydrogenation with lO% palladium on charcoal - (2OO mg) as catalyst at 4 atm for 3 hours. The catalyst was filtered off, washed with MeOH and the filtrate was concentrated to give the title compound (l.8 g, lOO%) as a white powder.

$R_f$ O.34 (methanol/triethylamine)

$^{13}$C-NMR ($CD_3OD$): δl73.8 (Cl); 76.5; 73.7; 7O.2; 68.l; 68.O (C2,4,5,6,7); 52.6 ($OCH_3$); 45.7 (C8); 29.6 (C3).

Example 4O Cbz-L-prolyl-L-methionine N-hydroxy-succinimide ester

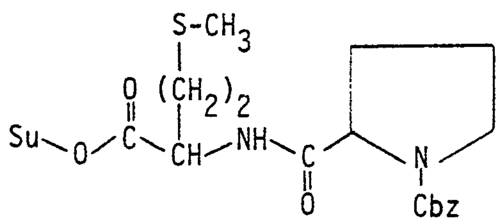

N-hydroxysuccinimide (O.l5 g, l.3 mmol) was added to a solution of Cbz-L-prolin-L-methionine (O.5 g, l.3 mmol) in dry THF (l5 ml). After cooling to O° DCC (O.27 g, l.3 mmol) was added with stirring. The mixture was kept in the refrigerator overnight. N,N'-dicyclohexylurea was filtered off and the solvent was evaporated in vacuo. Precipitation of the residue from MeOH/ether gave the title compound (O.56 g, 9O%).

$R_f$ O.5 (ethyl acetate)

$^{13}$C-NMR ($CDCl_3$): δl68.6; l67.5; l56 (carbonyls); l36.4; l28.6; l28.l; l27.9 (arom.); 67.4 ($CH_2Ph$); 6O (CH); 47 ($CH_2$); 32; 3O; 28; 26; 25 ($CH_2$); l5.4 ($SCH_3$).

Example 4l Methyl N-(Cbz-L-alanyl-L-phenylalanyl)-8-amino-2,6-anhydro-3,8-dideoxy-D-glycero-D-talo-octonate

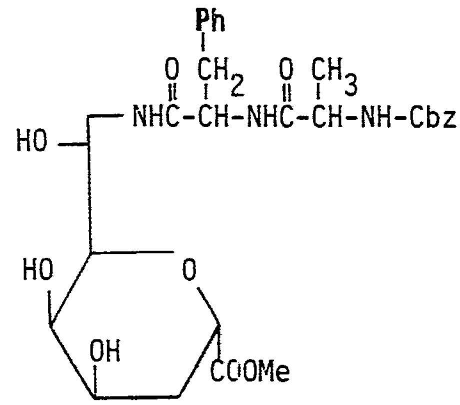

N-Cbz-L-alanyl-L-phenylalanine N-hydroxysuccinimide ester (600 mg, 1.3 mmol) and methyl 8-amino-2,6-anhydro-3,8-dideoxy-D-glycero-D-talo-octonate (270 mg, 1.1 mmol; Example 39) were dissolved in methanol/THF 10:1 (11 ml) and stirred at room temperature for 3 hours. The reaction mixture was concentrated and the residue was purified on silica gel with chloroform/methanol 8:1. This afforded the title compound (210 mg, 31%).

$R_f$ 0.35 (chloroform/methanol 8:1)

$^{13}$C-NMR (DMF-$d_7$): δ173.2; 173.1; 172.0; 156.9 (carbonyls); 138.7; 137.9; 130.0; 129.0; 128.7; 128.4; 126.8 - (arom.); 77.1; 73.0; 68.8; 67.3; 66.5; 55.0 (CH); 52.2 ($OCH_3$); 51.7 (CH); 44.2 (C8); 38.4 ($CH_2Ph$); 29.6 (C3); 18.3 ($CH_3$).

Example 42

Methyl N-(Cbz-L-prolyl-L-methionyl)-8-amino-2,6-anhydro-3,8-dideoxy-D-glycero-D-talo-octonate.

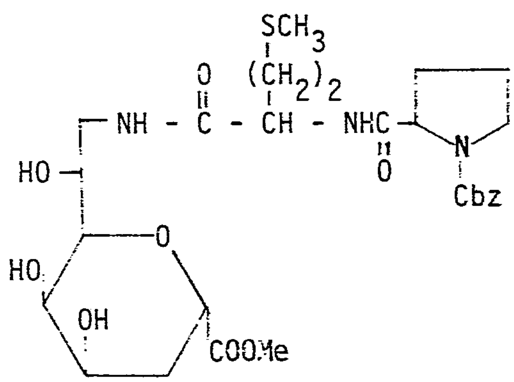

Cbz-L-pro-L-met-OSu(0.56 g, 1.17 mmol; Example 40) and methyl 8-amino-2,6-anhydro-3,8-dideoxy-D-glycero-D-talo-octonate (0.27 g, 1.15 mmol; Example 39) were dissolved in dry DMF and stirred at room temperature for 1 hour. The solvent was evaporated in vacuo and purification on silica gel with chloroform/methanol 9:1 afforded the title compound (0.36 g, 51%).

$R_f$ 0.5 (chloroform/methanol 8:1).

$^{13}$C-NMR($CDCl_3$): δ 172.9; 172.8; 172.5; 156.1 (carbonyl); 136.3; 128.6; 128.1; 127.8 (arom.); 75.5; 72.6; 68.6; 67.5 (CH); 66.7 ($CH_2$); 66,6; 61.0 (CH): 52.9 ($OCH_3$); 52.4 (CH); 47.3 ($CH_2$): 43.5 (C-8); 31.7; 30.5; 30.5: 29.0; 24.6 ($CH_2$); 15.4 ($SCH_3$)

Example 43

Methyl-N-(Cbz-L-lysyl(Cbz)-L-leucyl)-8-amino-2,6-anhydro-3,8-dideoxy-D-glycero-D-talo-octonate

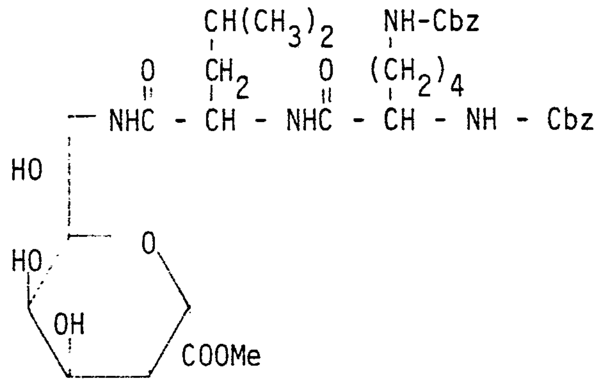

Cbz-L-lys(Cbz)-L-leu N-hydroxisuccinimide ester (570 mg, 0.9 mmol) and methyl 8-amino-2,6-anhydro-3,8-dideoxy-D-glycero-D-talo-octonate (190 mg, 0.8 mmol; Example 39) were dissolved in methanol (8 ml). After 2 hours at room temperature the solvent was evaporated in vacuo and purification on silica gel with chloroform/methanol 8:1 afforded the title compound (313 mg, 51 %).

$R_f$ 0.36 (chloroform/methanol 8:1).

$^{13}$C-NMR (DMF-$d_7$): $\delta$ 173.2; 173.1; 172.8; 157.1 (carbonyls); 138.3; 137.9; 129.0; 128.3 (arom); 77.1; 73.0; 68.8; 67.3; 67.2; 66.4; 66.0; 57.3; 56.1 (CH); 52.2 ($OCH_3$); 44.0; 41.7; 41.1 ($CH_2$); 30.1; 29.8; 29.5; 29.4; 25.1; 23.4; 21.8.

Example 44

Methyl N-(Cbz-L-alanyl-L-arginyl(di-Cbz))-8-amino-2,6-anhydro-3,8-dideoxy-D-glycero-D-talo-octonic acid.

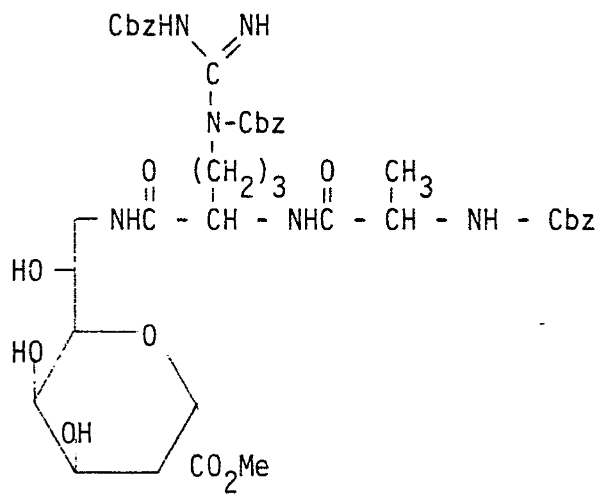

Cbz-L-ala-L-arg (di-Cbz) N-hydroxysuccinimide ester (0.84 g, 1.1 mmol) and methyl 8-amino-2,6-anhydro-3,8-dideoxy-D-glycero-D-talo-octonate (0.25 g, 1.1 mmol; Example 39) were dissolved in methanol - (12 ml) and triethylamine (0.2 ml, 1.4 mmol) was added. The reaction mixture was stirred at room temperature for 2 hours. The precipitate was filtered off and washed with methanol. Drying over $P_2O_5$ in vacuo afforded the title compound (370 mg, 39 %).

$R_f$ 0.6 (chloroform/methanol 4:1).

$^{13}$C-NMR (DMF-$d_7$): $\delta$ 173.4; 173.1; 172.4 (carbonyl); 164.1; 161.0; 156.2; 138.1; 137.8; 136.2; 129.2; 128.9; 128.8; 128.4; 128.3 (arom.); 77.1; 73.0; 69.1; 68.7; 67.3: 67.2; 67.0; 66.4; 53.6; 52.2; 51.5: 45.1: 44.1; 30.0; 29.5; 25.7; 18.4.

Example 45

Methyl N-(Cbz-L-leucyl-L-leucyl)-8-amino-2,6-anhydro-3,8-dideoxy-D-glycero-D-talo-octonate

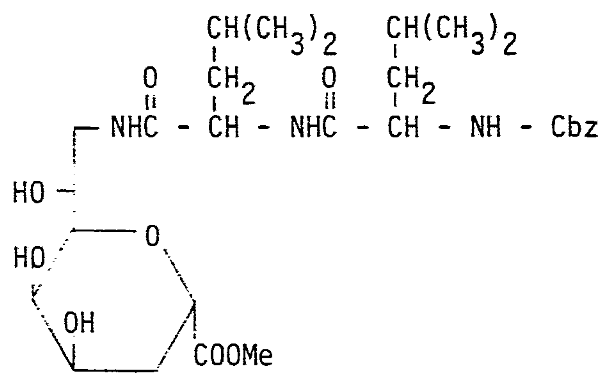

Cbz-L-leu-L-leu N-hydroxysuccinimide ester (570 mg, 1.2 mmol) and methyl 8-amino-2,6-anhydro-3,8-dideoxy-D-glycero-D-talo-octonate (250 mg, 1.1 mmol; Example 39) were dissolved in methanol/TMF 1:1 (8 ml). Stirring at room temperature overnight followed by concentration and precipitation from methanol/water/1:2 afforded the title compound (560 mg, 85 %).

$R_f$ 0.36 (chloroform/methanol) 8:1.

$^{13}$C-NMR (DMF-$d_7$): $\delta$ 173.3; 173.2; 172.9; 157.1 (carbonyls); 138.0; 129.0; 128.4; 128.2 (arom.); 77.0; 73.0; 68.8; 67.2; 66.5; 66.3; 54.5; 52.2 ($OCH_3$): 44.0; 41.7 ($CH_2$); 25.1; 23.3; 21.7.

## Example 46

Methyl N-L-norvalyl-8-amino-2,6-anhydro-3,8-dideoxy-D-glycero-D-talo-octonate

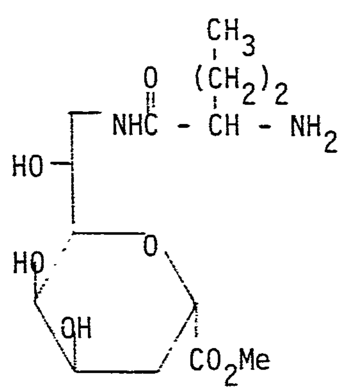

Cbz-L-norvaline N-hydroxysuccinimide ester (1.2 g, 3.4 mmol) and methyl 8-amino-2,6-anhydro-3,8-dideoxy D-glycero-D-talo-octonate (0.68 g, 2.9 mmol; Example 39) were dissolved in THF/methanol 1:1 (16 ml) and stirred at room temperature for 2 hours. After concentration in vacuo and precipitation from methanol/ether methyl N-Cbz-norvaline-8-amino-2,6-anhydro-3,8-dideoxy-D-glycero-D-talo-octonate was de-protected according to the general procedure E. Concentration of the filtrate afforded the title compound - (500 mg, 44 %).

$R_f$ 0.64 (methanol/triethylamine 9:1).

$^{13}$C-NMR (DMF-$d_7$): $\delta$ 174.0; 173.1 (carbonyl); 77.1; 73.0; 69.0; 67.4: 67.2; 55.6 (CH); 52.2 ($OCH_3$); 43.6 (C-8); 38.0 ($CH_2$); 29.6 (C-3); 19.4 ($CH_2$); 14.2 ($CH_3$).

## Example 47

N-(L-alanyl-L-phenylalanyl)-8-amino-2,6-anhydro-3,8-dideoxy-D-manno-L-glycero-octonic acid

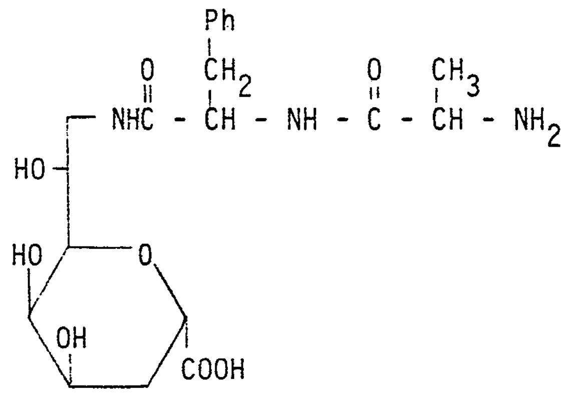

Methyl N-(Cbz-L-ala-L-phe)-8-amino-2,6-anhydro-3,8-dideoxy-D-glycero-D-talo-octonate (0.2l g, 0.36 mmol; Example 4l) was deprotected according to the general procedures B and C. Concentration of the filtrate afforded the title compound (l60 mg, 100 %).

$R_f$ 0.62 (2-propanol/water 3:2).

$^{13}$C-NMR ($D_2O$): δ 179.0; 174.0; 171.4 (carbonyl); 137.1; 130.3; 129.5 (arom.); 75.1; 74.9; 67.9; 67,7; 67.1; 56.6, 49.9 (CH); 43.5 (C-8); 38.0 ($CH_2Ph$): 29.5 (C-3); 17.2 ($CH_3$).

Example 48

N-(L-prolyl-L-methionyl)-8-amino-2,6-anhydro-3,8-dideoxy-D-glycero-D-talo-octonic acid

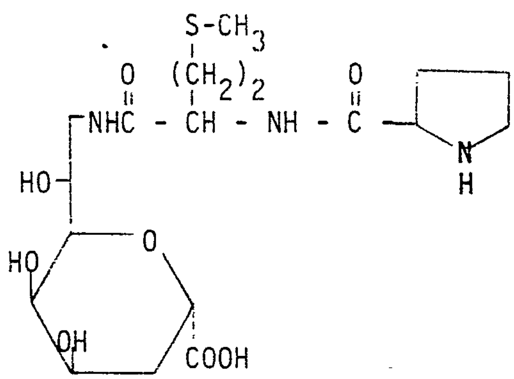

Methyl N-(Cbz-L-pro-L-met)-8-amino-2,6-anhydro-3,8-dideoxy-D-glycero-D-talo-octonate (0.33 g, 0.55 mmol; Example 42) was deprotected according to the general procedures B and C. Concentration of the filtrate afforded the title compound (145 mg; 57 %).

$R_f$ 0.56 (2-propanol/water 3:2)

$^{13}$C-NMR ($D_2O$): δ 179.1; 175.2; 171.3 (carbonyl); 76.4; 76.1; 69.2; 68.7; 68.2: 61.7; 55.7 (CH); 48.3 ($CH_2$) 44.6 (C-8); 32.4; 31.3; 31.2; 30.5; 25.5 ($CH_2$); 16.1 ($SCH_3$).

Example 49

N-(L-lysyl-L-leucyl)-8-amino-2,6-anhydro-3,8-dideoxy-D-glycero-D-talo-octonic acid

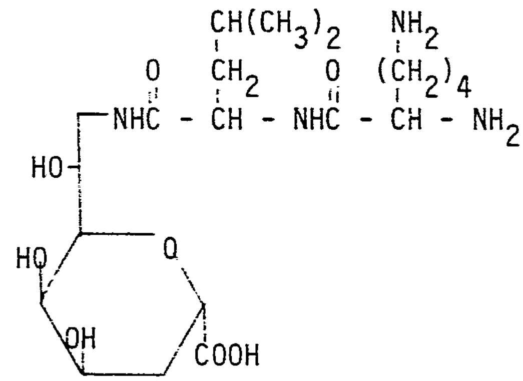

Methyl N-(Cbz-L-lys(Cbz)-L-leu)-8-amino-2,6-anhydro-3,8-dideoxy--D-glycero-D-talo-octonate (300 mg, 0.4 mmol; Example 43) was deprotected according to the general procedures A and C. Concentration of the filtrate afforded the title compound (140 mg, 76 %).

$R_f$ 0.1 (2-propanol/water 3:2).

$^{13}C$-NMR ($D_2O$): $\delta$ 179.1; 176.1; 176.0 (carbonyls); 75.5; 75.1; 68.3; 67.7; 67.2; 54.6; 54.0 (CH); 43.5; 41.0; 40.1 ($CH_2$); 33.5; 29.5; 27.4; 25.3; 23.0; 22.5; 21.8.

Example 50 N-(L-alanyl-L-arginyl)-8-amino-2.6-anhydro-3,8-dideoxy-D-glycero-D-talo-octonic acid

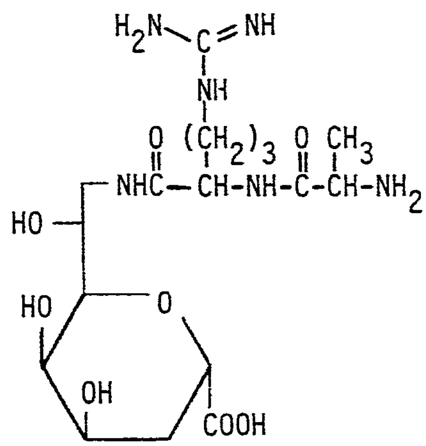

Methyl N-(Cbz-L-ala-L-arg(di-Cbz)-8-amino-2,6-anhydro-3,8-dideoxy-D-glycero-D-talo-octonic acid (365 mg, 0.42 mmol; Example 44) was deprotected according to the general procedures B and C. Concentration of the filtrate afforded the title compound (190 mg, 100%).

$^{13}C$-NMR ($D_2O$): $\delta$179.6; 176.6; 174.9 (carbonyls); 157.7; 75.2; 75.1; 67.9; 67.8; 67.1; 54.2; 50.5 (CH); 43.4; 41.0 ($CH_2$); 29.4; 29.2; 25.1 ($CH_2$); 19.6 ($CH_3$)

Example 51 N-(L-leucyl-L-leucyl)-8-amino-2,6-anhydro-3,8-dideoxy-D-glycero-D-talo-octonic acid

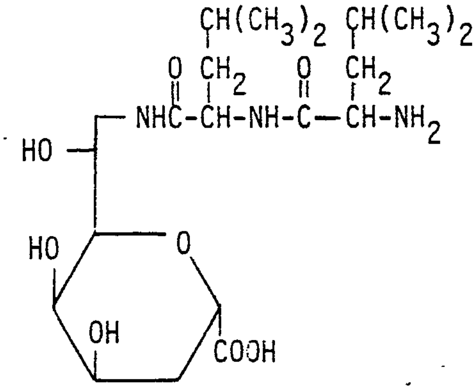

Method N(Cbz-L-leu-L-leu)-8-amino-2,6-anhydro-3,8-dideoxy-D-glycero-D-talo-octonate (340 mg,. 0.57 mmol; Example 45) was deprotected according to the general procedures A and C. Concentration of the filtrate afforded the title compound (230 mg, 90%)

$R_f$ 0.64 (2-propanol/water 3:2)

$^{13}C$-NMR ($CD_3OD$):δ178.9; 175.5; 171.4 (carbonyls); 76.1; 75.9; 68.9; 68.9; 54.8; 53.6 (CH); 43.9; 42.5; 42.0 ($CH_2$); 26.1; 25.6; 23.8; 23.5; 22.7; 22.5.

Example 52 Methyl N-(Cbz-L-arginyl(di-Cbz)-L-norvalyl)-8-amino-2,6-anhydro-3,8-dideoxy-D-glycero-D-talo-octonate

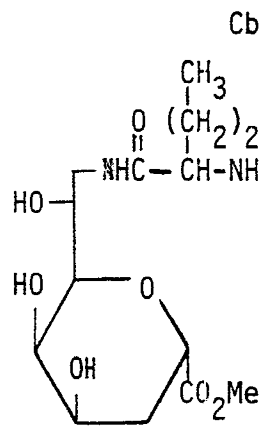

Cbz-L-arginine-(di-Cbz) N-hydroxysuccinimide ester (600 mg, 0.9 mmol) and methyl N-L-norvalyl-8-amino-2,6-anhydro-3,8-dideoxy-D-glycero-D-talo-octonate (300 mg, 0.9 mmol; Example 46) were dissolved in methanol/TMF 1:1 (40 ml) and stirred at room temperature for 2 hours. Evaporation of the solvent in vacuo and purification on silica gel with chloroform/methanol first 20:1 and then 8:1 afforded the title compound - (390 mg, 49%).

$R_f$ 0.39 (chloroform/methano 8:1)

$^{13}C$-NMR ($CDCl_3$ + $CD_3OD$): δ174.0; 173.3 (carbonyls); 135.4; 129.2; 128.9; 128.8; 128.5; 128.3; 128.2 (arom.); 76.0; 73.0; 69.5; 68.8; 67.7; 67.4; 67.0; 55.7; 54.0 (CH); 52.6 ($OCH_3$); 45.0; 43.4; 34.7; 29.1; 25.6; 19.3 ($CH_2$); 13.8 ($CH_3$).

Example 53 Methyl N-(Cbz-N-methyl-L-alanyl-L-norvalyl)-8-amino-2,6-anhydro-3,8-dideoxy-D-glycero-D-talo-octonate

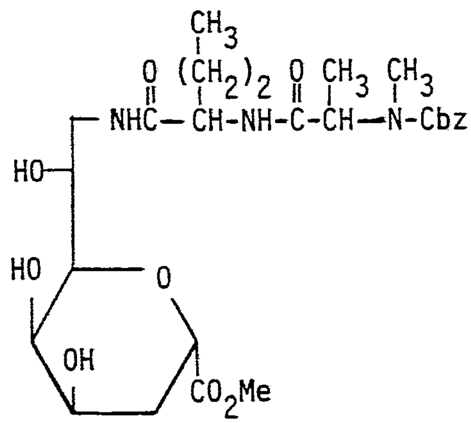

Methyl N-L-nva-8-amino-2,6-anhydro-3,8-dideoxy-D-glycero-D-talo-octonate (360 mg, l.l mmol; Example 46) and Cbz-N-methyl-L-alanine N-hydroxysuccinimide ester (385 mg, l.l mmol) were dissolved in methanol/THF l:l (20 ml) and stirred at room temperature for 3 hours. Concentration and purification on silica gel with chloroform/methanol 20:l afforded the title compound (200 mg, 33%).

$R_f$ = 0.3 (chloroform/methanol 8:l)

$^{13}$C-NMR ($CD_3OD$): δl74.l; l73.9 (carbonyls); l38.0; l29.5; l29.l; l28.l (arom.); 77.l; 73.8; 69.2; 68.6; 67.9; 56.2; 54.8 (CH); 52.9 ($OCH_3$); 44.l (C8); 35.2; 3l.l; 29.6; 20.l; l4.7; l4.0 ($CH_3$).

Example 54 Methyl N-(Cbz-L-valyl-L-methionyl)-8-amino-2,6-anhydro-3,8-dideoxy-D-glycero-D-talo-octonate

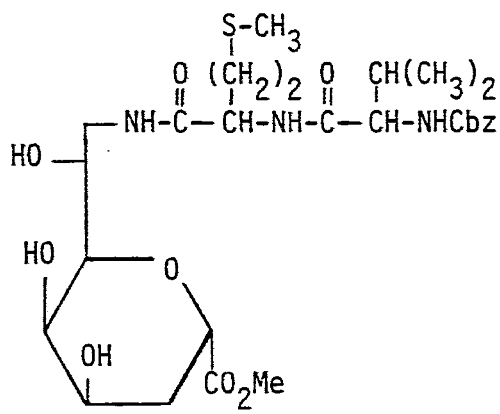

Cbz-L-valyl-L-methionine (287 mg, 0.75 mmol), methyl 8-amino-2,6-anhydro-3,8-dideoxy-D-glycero-D-talo-octonate (l50 mg, 0.64 mmol; Example 39) l-hydroxybenzotriazole (70 mg, 0.5 mmol), triethylamine - (97 µl, 0.7 mmol) and dicyclohexyl carbodiimide (l65 mg, 0.8 mmol) were dissolved in dry DMF (5 ml) and stirred at room temperature overnight. Concentration and purification on silica gel with chloroform/methanol l0:l afforded the title compound l75 mg, 20%.

$R_f$ 0.4 (chloroform/methanol 8:l).

$^{13}$C-NMR (DMF-$d_7$): δl73.3; l72.6; l72.5 (carbonyls); l38.l; l29.l; l28.5; l28.4 (arom.); 77.2; 73.2; 68.7; 67.4; 67.3; 66.7; 6l.6; 53.2 (CH); 52.3 ($OCH_3$); 44.0 (C8); 32.7; 30.7; 29.6; 26.3; 25.5; l9.7; l8.3; l5.0 ($CH_3$).

Example 55 Methyl N-DL-2-aminopimelyl-8-amino-2,6-anhydro-3,8-dideoxy-D-glycero-D-talo-octonate

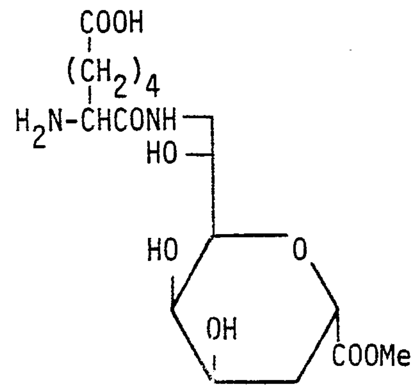

Methyl 8-amino-2,6-anhydro-3,8-dideoxy-D-glycero-D-talo-octonate (0.823 g, 3.5 mmol; Example 39) and triethylamine (0.25 ml) were dissolved in MeOH (7 ml). The mixture was cooled to 0°C and DL-2,5-dioxooxazolidine-4-pentanoic acid (0.30 g, 1.5 mmol), prepared according to J. Med. Chem. 1986 29, 89, was added in one portion. After stirring for 30 min, solvents were evaporated and the residue was purified on an anion exchange resin in the carbonate form. Elution with MeOH (30 ml) was followed by a solution of triethylammonium hydrogen carbonate (5%) in MeOH. The fractions containing a ninhydrin-sensitive spot of $R_f$ 0.6 on TLC in 2-propanol-$H_2O$ (3:2) were collected to give 0.35 g of the title compound. It exhibited a $^1H$-NMR spectrum in agreement with the above structure.

Example 56 N-(L-arginyl-L-norvalyl)-8-amino-2,6-anhydro-3,8-dideoxy-D-glycero-D-talo-octonic acid

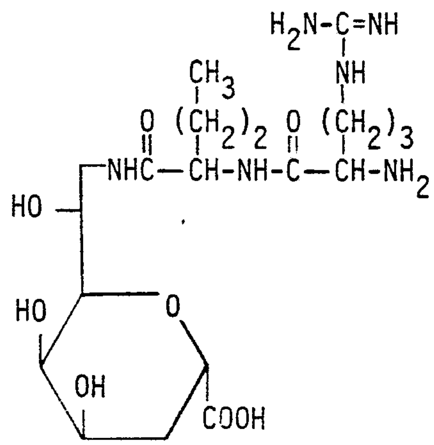

Methyl N-(Cbz-L-arg(di-Cbz)-L-nva)-8-amino-2,6-anhydro-3,8-dideoxy-D-glycero-D-talo-octonate (347 mg, 0.39 mmol; Example 52) was deprotected according to the general methods A and D. Concentration of the filtrate afforded the title compound (150 mg, 81%).

$^{13}C$-NMR ($D_2O$): δ178.7; 175.2 (carbonyls); 157.5; 75.2; 74.9; 68.2; 67.5; 67.1; 54.9; 54.3 (CH); 43.3; 41.4; 34.2; 30.6; 29.4; 24.7; 19.2 ($CH_2$); 13.7 ($CH_3$)

Example 57 N-(N-methyl-L-alanyl-L-norvalyl-8-amino-2,6-anhydro-3,8-dideoxy-D-glycero-D-talo-octonic acid

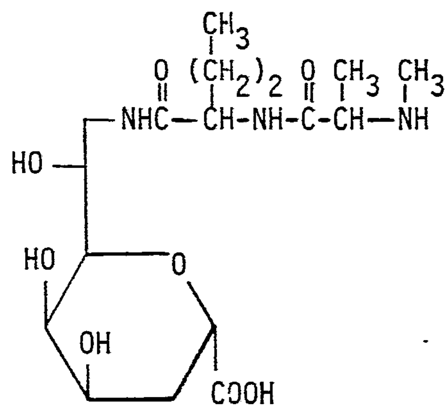

Methyl N-(Cbz-N-Me-L-ala-L-nva)-8-amino-2,6-anhydro-3,8-dideoxy-D-glycero-D-talo-octonate (200 mg, 0.36 mmol; Example 53) was deprotected according to the general procedures A and D. Concentration of the filtrate afforded the title compound (100 mg, 69%).

$R_f$ 0.62 (2-propanol/water 3:2)

$^{13}$C-NMR ($D_2O$): δ179.2; 175.1; 171.7 (carbonyls); 75.4; 75.1; 68.2; 67.7; 67.2; 58.1; 55.3 (CH); 43.5 (C8); 34.2 ($CH_2$); 32.2 ($NHCH_3$); 29.5 (C3); 19.4 ($CH_2$); 16.3; 13.8 ($CH_3$)

Example 58 N-(L-valyl-L-methionyl)-8-amino-2,6-anhydro-3,8-dideoxy-D-glycero-D-talo-octonic acid

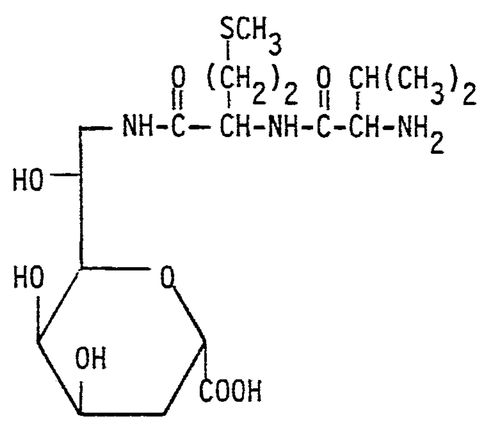

Methyl N-(Cbz-L-val-L-met)-8-amino-2,6-anhydro-3,8-dideoxy-D-glycero-D-talo-octonate (96 mg, 0.16 mmol; Example 54) was deprotected according to general procedures A and D. Concentration of the filtrate afforded the title compound (50 mg, 68%).

$R_f$ 0.38 (2-propanol/water 3:2)

$^{13}$C-NMR ($D_2O$): δ179.1; 174.3; 171.5 (carbonyls); 75.4; 75.1; 68.2; 67.7; 67.2; 59.7; 54.6 (CH); 43.5 (C8); 31.5; 31.1; 30.2; 29.5; 18.8; 17.6; 15.1 ($CH_3$).

Example 59 Dimethyl N-[Cbz-L-alanyl-DL-2-amino-(6-methoxycarbonyl)-hexanoyl]-8-amino-2,6-anhydro-3,8-dideoxy-D-glycero-D-talo-octonate

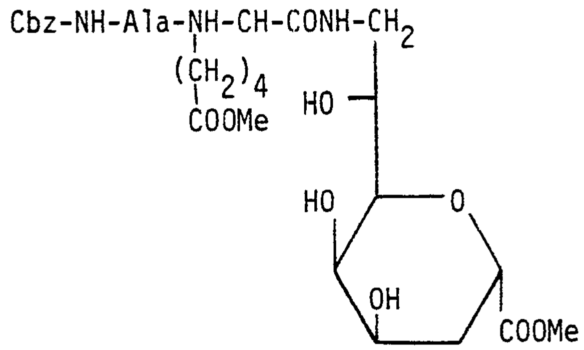

The compound from Example 55 (0.37 g, 0.94 mmol) was dissolved in MeOH (10 ml) and triethylamine (0.2 ml) was added followed by Cbz-L-alanine N-hydroxysuccinimide ester (0.38 g, 1.2 mmol). After 20 h the mixture was concentrated in vacuum and dry DMF (5 ml) was added followed by potassium carbonate - (0.42 mg, 3.0 mmol) and methyl iodide (0.71 g, 5 mmol). The mixture was stirred for 2 days, concentrated and the residue was chromatographed on a silica gel column using $CHCl_3$-MeOH (10:1) as an eluent. Concentration of fractions containing the product gave 0.26 g of product consisting of two compounds having very close $R_f$ values of 0.39 and 0.35 in $CHCl_3$-MeOH (8:1).

$^1$H-NMR ($CDCl_3$) for selected peaks: 7.35 (s,SH); 5.1 (brs, 2H); 1.35 (two d, 3H).

$^{13}$C-NMR: 174.2; 173.2; 172.7; 172.6; 136.1; 128.3; 127.9; 127.7; 75.1; 72.3; 68.1; 66.8; 66.4; 53.1; 52.2; 51.3; 50.6; 48.7; 42.7; 33.5; 31.6; 28.5; 24.8; 24.2; 18.0.

Example 60 Ammonium N-(L-alanyl-DL-2-aminopimelyl)-8-amino-2,6-anhydro-3,8-dideoxy-D-glycero-D-talo-octonate

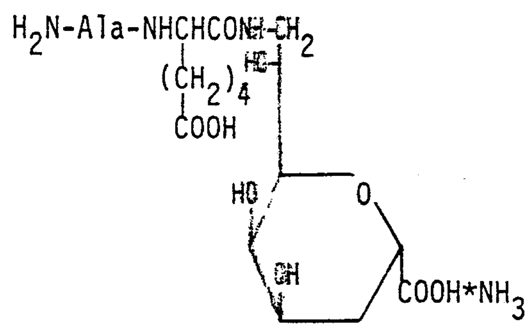

The compound (0.25 g, 0.41 mmol) from Example 59 was deprotected according to General Procedures A and D to yield the title compound (0.17 g). This was further purified on a column of microcrystalline cellulose using acetonitrile-$H_2O$-MeOH (2:1:1) as an eluent. Concentration of fractions containing ninhydrin-sensitive material at $R_f$ 0.6 on a cellulose plate gave 123 mg of the title compound.

$^{13}$C-NMR ($D_2O$): δ(dioxane): 182.8 181.8; 179.0; 75.7; 75.0; 68.4; 67.6; 67.3; 55.6; 52.0; 43.5; 37.1; 32.4; 29.4; 25.9; 25.7; 19.1.

Example 61 Benzyl 4,5,7-O-benzyl-8-O-(Cbz-L-alanyl-L-alanyl-N-methyl-glycyl)-2,6-anhydro-3-deoxy-O-glycero-D-talo-octonate

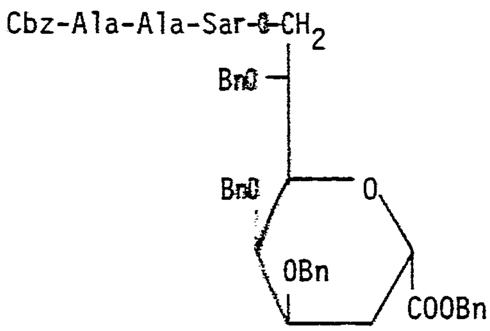

Cbz-Ala-Ala-Sar-OH (161 mg, 0.44 mmol), prepared from Cbz-Ala-Ala and sarcosine t-butyl ester by standard methods, was mixed with the alcohol from example 10 (200 mg, 0.34 mmol) in 5 ml of dichloromethane. DCC (103 mg, 0.5 mmol) and 4-dimethylaminopyridine (10 mg) were added to the ice-cooled solution. After 2 h 5 drops of $H_2O$ and $KHCO_3$ (30 mg) were added and after another 0.5 h $Na_2SO_4$ was added for drying. Filtration, evaporation of solvents and chromatography on silica gel using ethyl acetate-hexane (1:1) as an eluent, yielded 201 mg (64%) of the title compound.

$R_f$ 0.41 (EtOAc-Hex, 3:2)

$^{13}C$-NMR ($CDCl_3$) exhibited the following selected peaks: δ172.7; 171.3; 171.2; 168.7; 155.7; 51.1; 50.8; 49.5; 45.5; 36.0; 27.2; 19.1; 18.3.

Example 62 8-O-(L-Alanyl-L-alanyl-N-methylglycyl)-2,6-anhydro-3-deoxy-D-glycero-D-talo-octonic acid

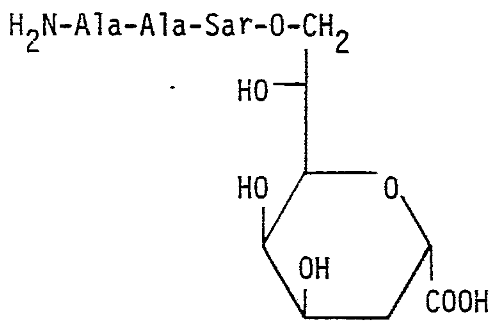

The compound from Example 61 (100 mg, 0.11 mmol) was hydrogenolyzed in acetic acid containing ammonium acetate (15 mg) and Pd on charcoal (100 mg). Filtration and freeze-drying gave 42 mg of material. Both $^{13}C$ and $^{1}H$-NMR indicated the expected product which, however, was not pure.

## Claims

1. A compound of the formula

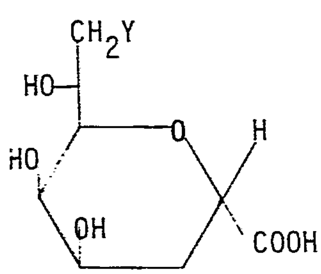

I

wherein Y is hydrogen, halogen such as fluoro or chloro, amino, acylamino, azido, mercapto, thiocarboxy,

acyloxy,

or a group -O-CHCOW (i)
NHAA

or a group -S-CHCOW (ii)
NHAA

wherein AA is an amino acid or a peptide sequence containing 2-4 amino acid residues and W is hydroxy, amino, benzyloxy or an amino acid or peptide sequence containing 2-4 amino acid residues; or a physiologically acceptable salt thereof.

2. A compound according to claim I, wherein Y is an acylamino group, where the acyl residue is an amino acid or peptide sequence, with 2 to 4 amino acid residues: or physiologically acceptable salts thereof.

3. A compound according to the claims I and 2 wherein Y is an acylamino group -NHCOR$^a$ wherein R$^a$CO-is an amino acid or an amino acid sequence containing 2-4 amino acid residues whereby the amino acid and the amino acid residue are independently selected from glycyl, histidyl, arginyl methionyl, alanyl, phenylalanyl, lysyl, ornithyl, norvalyl, valyl, norleucyl, isoleucyl, leucyl, prolyl or 2-aminopimelyl, either in the L-or in the D-configuration; or physiologically acceptable salts thereof.

4. A compound according to claim I wherein Y is

a group -O-CHCOW
NHAA

or a group -S-CHCOW (ii)
NHAA

wherein AA is an amino acid or a peptide sequence containing 2-4 amino acid residues chosen from the group consisting of glycyl, norvalyl, alanyl, phenylalanyl, histidyl, lysyl, ornithyl, valyl, leucyl, arginyl, norleucyl, isoleucyl, methionyl, prolyl or 2-aminopimelyl, either in the L-or in the D-configuration and W is hydroxy, amino, benzyloxy or an amino acid or peptide sequence containing 2-4 amino acid residues chosen from the group above either in the L-or in the D-configuration; or a physiologically acceptable salt thereof.

5. A compound according to one of the claims I-4 in form of a physiologically acceptable salt.

6. A compound according to claim 5 in form of an ammonium salt.

7. A pharmaceutical preparation comprising as an active ingredient a compound according to any of claims I-6 or a physiologically acceptable salt thereof.

8. A pharmaceutical preparation according to claim 7 comprising the active ingredient in association with a pharmaceutical carrier.

9. A pharmaceutical preparation according to claim 7 designed for systemic administration.

IO. A pharmaceutical preparation according to claim 7 in dosage unit form.

II. A compound according to any of claims I-6 for use as a drug.

I2. A compound according to any of claims I-6 for use as a drug for treatment of infectious diseases.

I3. The use of a compound according to any of the claims I-4 or a physiologically acceptable salt thereof for preparation of a pharmaceutical preparation comprising as an active ingredient an amount of said compound for the treatment of infectious diseases.

I4. A method for the treatment of bacterial infections in an animal or human host in need of such treatment, comprising administering a therapeutically effective amount of a compound according to any of the claims I-6.

I5. A process for preparing a compound of the formula

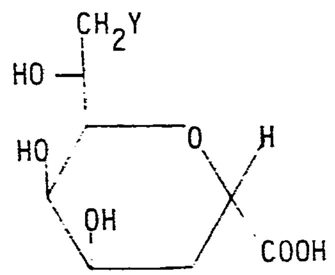

I

wherein Y is hydrogen, halogen such as chloro or fluoro, amino, acylamino, azido, acyloxy, mercapto, thiocarboxy,

or a group -O-CHCOW (i)
NHAA

or a group -S-CHCOW (ii)
NHAA

wherein AA is an amino acid or a peptide sequence containing 2-4 amino acid residues and W is hydroxy, amino, benzyloxy or an amino acid or peptide sequence containing 2-4 amino acid residues; or a physiologically acceptable salt thereof
characterized in that

A. Reacting a compound of the formula

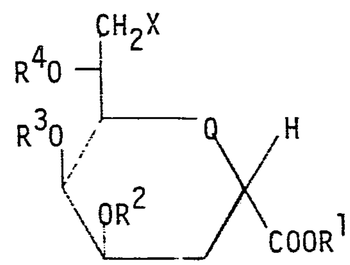

with a nucleophilic reagent followed by removal of optional protecting groups in which formula X is a leaving group, $R^2$-$R^4$ are the same or different and selected from hydrogen or a protecting groups such as acyl, allyl, tetrahydropyranyl, silyl or cyclic acetal, $R^1$ is hydrogen or a protecting group such as alkyl containing l-6 carbon atoms, benzyl or allyl, leading to information of a compound of formula I, wherein Y is halogen, amino, acyloxy, azido, mercapto or thiocarboxy;

B. Reacting a hydroxy-ester

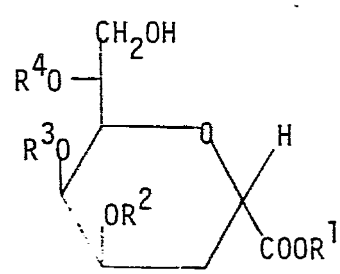

in which formula $R^1$-$R^4$ are as defined under A; with a phosphorus-or sulfur-based electrophilic reagent followed by removal of optional protecting groups, leading to formation of a compound of formula I wherein Y is fluoro, chloro, thiocarboxy or azido;

C. Reductive amination of an aldehyde of the formula

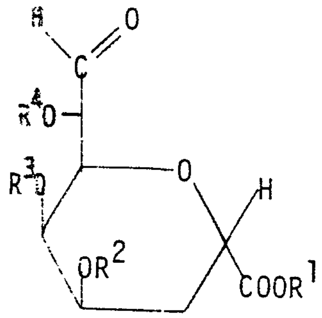

in which formula $R^1$-$R^4$ are protecting groups as defined under A with an amine $H_2N$-$R^9$ wherein $R^9$ is hydrogen, benzyl or allyl followed by removal of protecting groups to formation of a compound of formula I wherein Y is amino;

D. Reacting an amine of the formula

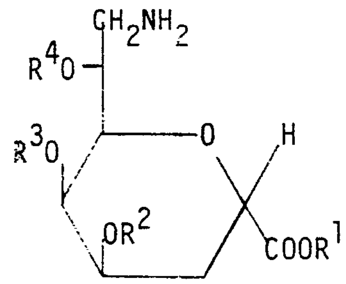

with an amino acid or amino acid sequence with the formula

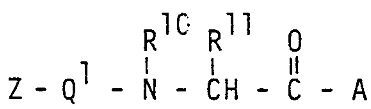

to formation of a compound of the formula

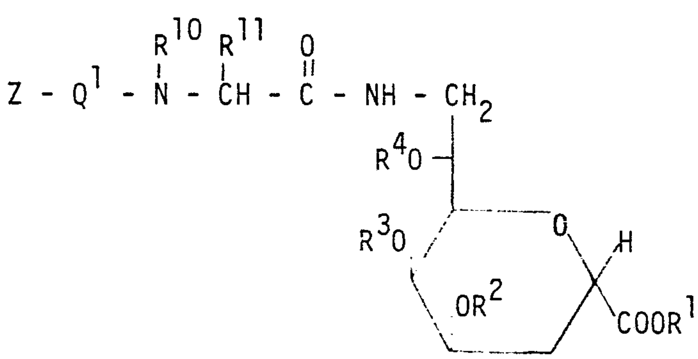

followed by removal of protecting groups, in which formulas $R^1$-$R^4$ are as defined under A: $R^{11}$ is lower alkyl, ω-amino lower alkyl, benzyl or 2-thioethyl: $R^{10}$ is lower alkyl or $R^{10}$ and $R^{11}$ together form a cyclic derivative containing 2-5 carbon atoms;

A is hydroxy or a carboxyl-activating group;

Z is an amino-protecting group;

$Q^1$ is a group containing from O-3 units of an optionally protected amino acid residue such as glycyl, alanyl, phenylalanyl, lysyl, ornithyl, norvalyl, valyl, arginyl, norleucyl, isoleucyl, histidyl, leucyl, methionyl, prolyl or 2-aminopimelyl.

E. Reacting an azide of the formula

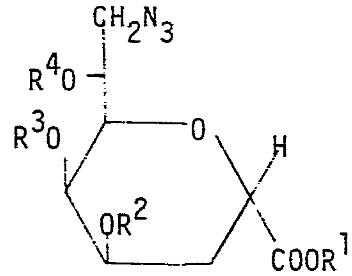

with an amino acid or amino acid sequence of the formula

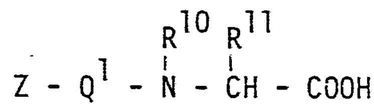

to formation of a compound of the formula

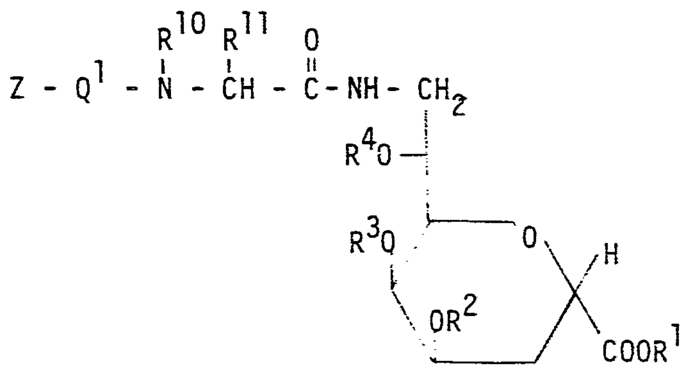

followed by removal of protecting groups, in which formulas $R^1$-$R^4$ are as defined under A; $R^{10}$, $R^{11}$, Z, $Q^1$ are as defined under D;

F. Reacting a hydroxy ester or thiol of the formula

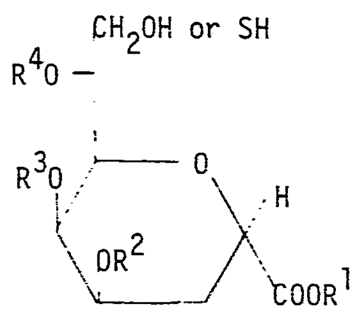

with a glycine derivative of the formula

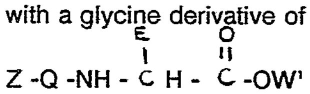

to formation of a compound of the formula

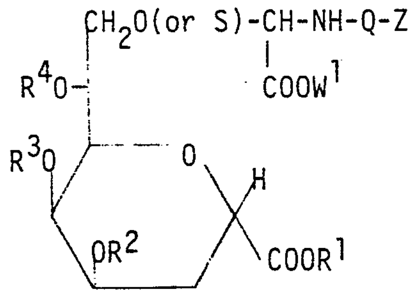

followed by removal of protecting groups, in which formulas $R^1$-$R^4$ are as defined under A; Z is defined under D; Q is a group containing l-4 units of an amino acid residue defined under D; $W^1$ is amino, benzyloxy or a protected amino acid or peptide sequence containing 2-4 amino acid residues; whereupon a compound obtained is optionally converted into a physiologically acceptable salt.

16. A chemical intermediate of the formula

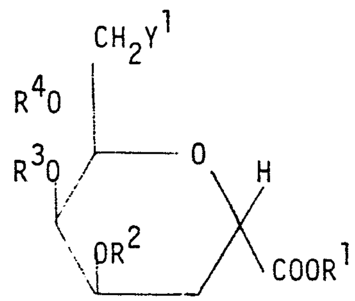

wherein $R^2$-$R^4$ are the same or different and selected from hydrogen or protecting groups such as tert. butyldimethylsilyl, benzyl;

$R^1$ is a protecting group such as benzyl or allyl. $Y^1$ is hydrogen, a halogen such as fluoro or chloro, amino, azido, mercapto, acylamino, thiocarboxy, acyloxy.

17. A chemical intermediate according to claim 16 wherein $Y^1$ is azido.

18. A compound which after administration to a living organism is transformed into a compound

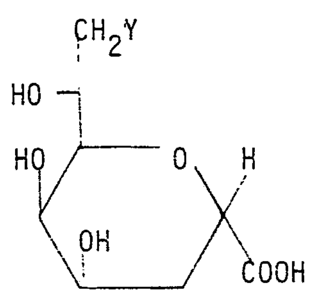

I

wherein Y is hydrogen, a halogen such as chloro or fluoro, amino, azido or mercapto and exerting its effect in this structural form.

19. A compound according to claim 18 wherein Y is amino.

20. Compounds, processes, pharmaceutical preparations and methods of treatment as claimed in any of the claims 1-19 and substantially as described.

European Patent Office

# PARTIAL EUROPEAN SEARCH REPORT

which under Rule 45 of the European Patent Convention shall be considered, for the purposes of subsequent proceedings, as the European search report

Application number
86850438.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| DX | ADVANCES IN CARBOHYDRATE CHEMISTRY AND BIOCHEMISTRY, vol. 38, 1981, pages 323-388 F.M. UNGER: "The chemistry and biological significance of 3-deoxy-D-manno-2-octulosonic acid (KDO)". * Pages 339-340; page 356 compounds 57,60; page 376 compounds 118-120; pages 365-388 * | 1-13, 15-17, 20 | C 07 H 7/02<br>C 07 H 9/04<br>C 07 H 13/02<br>C 07 H 15/04<br>C 07 K 9/00<br>A 61 K 31/70 |
| DX | AMERICAN CHEMICAL SOCIETY SYMPOSIUM SERIES 231, 1983, pages 141-169, Washington DC; P.H. RAY et al.: "Synthesis and use of 3-deoxy-D--manno-2-octulosonate (KDO) in Escherichia coli. Potential sites of inhibition". * Pages 141 and 154-156, "Discussion" pages 164-168 * | 1-13, 15-17, 20 | |
| A | CHEMICAL COMMUNICATIONS, No. 21, 1981, pages 1139-1140, London; P.M. COLLINS et al.: "Synthesis of 3-deoxy-D-manno-octulosonic acid (KDO)" | 1-13, 15-17, 20 | TECHNICAL FIELDS SEARCHED (Int. Cl.4)<br>C 07 H<br>C 07 K<br>A 61 K |

- / -

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched: 14, 18, 19 and 20(in part)

Reason for the limitation of the search:

Claims 14 and 20(in part): Method for treatment of the human or animal body by therapy.

Claims 18, 19 and 20(in part): The compounds covered by these claims are not specified. Only the compounds formed from the unspecified compounds are given.

| Place of search | Date of completion of the search | Examiner |
|---|---|---|
| STOCKHOLM | 17-03-1987 | CLAESSON G. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number

86850438.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CANADIAN JOURNAL OF BIOCHEMISTRY, vol. 47, no. 7, 1969, pages 691-695; D.T. WILLIAMS et al.: "The structure, synthesis, analysis, and occurrence of 3-deoxy-D-manno-octulosonic acid". ----- | 1-13, 20 | |
| A | EP-A-0 160 925 (KANTO ISHI PHARMACEUTICAL CO., LTD) ----- | 1 | |
| P | US-A-4 613 589 (ABBOTT LABORATORIES) ----- | 1-13, 15-17,20 | |
| P | US-A-4 613 590 (ABBOTT LABORATORIES) ----- | 1-13, 15-17,20 | TECHNICAL FIELDS SEARCHED (Int. Cl.4) |